# EUROPEAN PATENT APPLICATION

(11) **EP 3 026 589 A1**
(43) Date of publication of application: **01.06.2016**
(21) Application number: 15196560.5
(22) Date of filing: 26.11.2015
(51) Int. Cl.: G06F 19/00

(54) **EXERCISE INFORMATION PROVIDING METHOD AND ELECTRONIC DEVICE SUPPORTING THE SAME**

(30) Priority: 26.11.2014 KR 20140166731
(71) Applicant: Samsung Electronics Co., Ltd., Gyeonggi-do 16677 (KR)
(72) Inventor: Ko, Kwang Won, 07258 Seoul (KR); Heo, Jun Seok, 06624 Seoul (KR); Park, Seung Hoon, 02855 Seoul (KR); Lee, Yu Won, 16708 Suwon-si (KR)
(74) Representative: Nederlandsch Octrooibureau

(57) **Abstract**

An electronic device and method are disclosed, the electronic device including an output module, a communication module configured to allow communication with at least one of a first electronic device and an external electronic device, and at least one processor, which implements the method, including receiving exercise portion information from a first electronic device, receiving exercise amount information from a second electronic device, and determining exercise information including a respective amount of exercise for each exercise portion based on at least the received exercise portion information and the received exercise amount information, and controlling the output module to output the exercise information.

## Description

### PRIORITY

This application claims priority to Korean Patent Application No. 10-20 14-0166731, filed November 26, 2014, the content of which is incorporated herein by r eference.

### BACKGROUND OF THE INVENTION

### 1. Field of the Invention

The present disclosure relates to an exercise information providing meth od and electronic device implementing the same.

### 2. Background of the Invention

As an interest in health is increased recently, as a method of maintaining good health, an interest in exercise is increased also. According to this trend, demand f or exercise based health management service, that is, exercise service, has increased an d the number of electronic devices with functions for supporting has correspondingly in creased.

However, electronic devices often collect results in relation to a digital s ervice and provide user information related to the digital service. Additionally, existing electronic devices may not reflect various internal/external conditions because they are executed by user's explicit instructions.

### SUMMARY OF THE INVENTION

Accordingly, an aspect of the present disclosure is to provide a method of integrating and analyzing a variety of information in real time through collaboration between electronic devices and an electronic device supporting the same.

Another aspect of the present disclosure is to provide a method of integr ating and analyzing information by using the so-called "Internet of Things" (IoT) for ex changing information through communication between electronic devices without user' s intervention and an electronic device supporting the same.

In one aspect of the present disclosure, an electronic device is disclosed, including an output module, a communication module configured to allow communicat ion with at least one of a first electronic device and a second electronic device, and at le ast one processor, configured to receive exercise portion information from the first elect ronic device, receive exercise amount information from the second electronic device, a nd determine exercise information including a respective amount of exercise for each e xercise portion based on at least the received exercise portion information and the recei ved exercise amount information.

In another aspect of the present disclosure, a method in an electronic dev ice is disclosed, including receiving via a communication module exercise portion infor mation from a first electronic device, receiving exercise amount information from a sec ond electronic device, determining by at least one processor exercise information inclu ding a respective amount of exercise for each exercise portion based on at least the rece ived exercise portion information and the received exercise amount information, and co ntrolling an output module to output the exercise information.

Other aspects, advantages, and salient features of the disclosure will bec ome apparent to those skilled in the art from the following detailed description, which, t aken in conjunction with the annexed drawings, discloses various embodiments of the p resent disclosure.

### BRIEF DESCRIPTION OF THE DRAWINGS

The above and other aspects, features, and advantages of certain embodi ments of the present disclosure will be more apparent from the following description ta ken in conjunction with the accompanying drawings, in which:
FIG. 1 is a view illustrating an exercise information providing system ac cording to various embodiments of the present disclosure;
FIG. 2 is a view illustrating an electronic device in an exercise informati on providing system according to various embodiments of the present disclosure;
FIG. 3 is a view illustrating an IoT based exercise information providing system according to various embodiments of the present disclosure;
FIG. 4A is a first view illustrating an exercise portion determination and exercise amount measurement on the basis of specified information according to variou s embodiments of the present disclosure;
FIG. 4B is a second view illustrating an exercise portion determination a nd an exercise amount measurement on the basis of specified information according to various embodiments of the present disclosure;
FIG. 4C is a third view illustrating an exercise portion determination and an exercise amount measurement on the basis of specified information according to var ious embodiments of the present disclosure;
FIG. 5 is a view illustrating an exercise portion determination and exerci se amount measurement on the basis of sensor information according to various embodi ments of the present disclosure;
FIG. 6 is a view illustrating an exercise portion determination and exerci se amount measurement on the basis of user input information according to various em bodiments of the present disclosure;
FIG. 7A is a first view illustrating an exercise portion determination and an exercise amount measurement on the basis of a plurality of information according to various embodiments of the present disclosure;
FIG. 7B is a second view illustrating an exercise portion determination a nd an exercise amount measurement on the basis of a plurality of information according to various embodiments of the present disclosure;
FIG. 7C is a third view illustrating an exercise portion determination and an exercise amount measurement on the basis of a plurality of information according t o various embodiments of the present disclosure;
FIG. 8 is a view illustrating an IoT based exercise information providing system according to various embodiments of the present disclosure;
FIG. 9 is a view illustrating an output screen of an exercise information providing device according to various embodiments of the present disclosure;
FIG. 10 is a view illustrating a method of operating an exercise informat ion providing system relating to an individual exercise according to various embodimen ts of the present disclosure;
FIG. 11 is a view illustrating a method of operating an exercise informat ion providing system relating to exercise service according to various embodiments of t he present disclosure;
FIG. 12 is a view illustrating a method of operating an IoT based exercis e information providing system according to various embodiments of the present disclo sure;
FIG. 13 is a view illustrating an electronic device in a network environm ent according to various embodiments of the present disclosure;
FIG. 14 is a block diagram illustrating an electronic device according to various embodiments of the present disclosure; and
FIG. 15 is a block diagram illustrating a program module according to v arious embodiments of the present disclosure.

Throughout the drawings, it should be noted that like reference numbers are used to depict the same or similar elements, features, and structures.

### DETAILED DESCRIPTION OF EMBODIMENTS OF THE PRESENT

### INVENTION

Hereinafter, various embodiments of the present disclosure are disclosed with reference to the accompanying drawings. However, this does not limit various em bodiments of the present disclosure to a specific embodiment and it should be understo od that the present disclosure covers all the modifications, equivalents, and/or alternativ es of this disclosure provided they come within the scope of the appended claims and th eir equivalents. With respect to the descriptions of the drawings, like reference numeral s refer to like elements.

The term "include," "comprise," and "have", or "may include," or "may comprise" and "may have" used herein indicates disclosed functions, operations, or exi stence of elements but does not exclude other functions, operations or elements.

For instance, the expression "A or B", or "at least one of A or/and B" m ay indicate include A, B, or both A and B. For instance, the expression "A or B", or "at least one of A or/and B" may indicate (1) at least one A, (2) at least one B, or (3) both a t least one A and at least one B.

The terms such as "1st" "2nd", "first", "second", and the like used herei n may refer to modifying various different elements of various embodiments of the pres ent disclosure, but do not limit the elements. For instance, "a first user device" and "a s econd user device" may indicate different users regardless of the order or the importanc e. For example, a first component may be referred to as a second component and vice v ersa without departing from the scope of the present disclosure.

In various embodiments of the present disclosure, it will be understood t hat when a component (for example, a first component) is referred to as being "(operati vely or communicatively) coupled with/to" or "connected to" another component (for e xample, a second component), the component may be directly connected to the other co mponent or connected through another component (for example, a third component). In various embodiments of the present disclosure, it will be understood that when a comp onent (for example, a first component) is referred to as being "directly connected to" or "directly access" another component (for example, a second component), another com ponent (for example, a third component) does not exist between the component (for exa mple, the first component) and the other component (for example, the second compone nt).

The expression "configured to" used in various embodiments of the pres ent disclosure may be interchangeably used with "suitable for", "having the capacity to" , "designed to", "adapted to", "made to", or "capable of" according to a situation, for ex ample. The term "configured to" may not necessarily mean "specifically designed to" i n terms of hardware. Instead, the expression "a device configured to" in some situations may mean that the device and another device or part are "capable of". For example, "a processor configured to perform A, B, and C" in a phrase may mean a dedicated proces sor (for example, an embedded processor) for performing a corresponding operation or a generic-purpose processor (for example, a CPU or application processor) for performi ng corresponding operations by executing at least one software program stored in a me mory device.

Terms used in various embodiments of the present disclosure are used to describe specific embodiments of the present disclosure, and are not intended to limit t he scope of other embodiments. The terms of a singular form may include plural forms unless they have a clearly different meaning in the context. Otherwise indicated herein, all the terms used herein, which include technical or scientific terms, may have the sam e meaning that is generally understood by a person skilled in the art. In general, the ter ms defined in the dictionary should be considered to have the same meaning as the cont extual meaning of the related art, and, unless clearly defined herein, should not be unde rstood abnormally or as having an excessively formal meaning. In any cases, even the t erms defined in this specification cannot be interpreted as excluding embodiments of th e present disclosure.

According to various embodiments of the present disclosure, electronic devices may include at least one of smartphones, tablet personal computers (PCs), mobi le phones, video phones, electronic book (e-book) readers, desktop personal computers (PCs), laptop personal computers (PCs), netbook computers, workstation server, person al digital assistants (PDAs), portable multimedia player (PMPs), MP3 players, mobile medical devices, cameras, and wearable devices (for example, smart glasses, head-mou nted-devices (HMDs), electronic apparel, electronic bracelets, electronic necklaces, ele ctronic appcessories, electronic tattoos, smart mirrors, and smart watches).

According to some embodiments of the present disclosure, an electronic device may be smart home appliances. The smart home appliances may include at least one of, for example, televisions, digital video disk (DVD) players, audios, refrigerators, air conditioners, cleaners, ovens, microwave ovens, washing machines, air cleaners, set -top boxes, home automation control panels, security control panels, TV boxes (e.g., Sa msung HomeSyncTM, Apple TVTM or Google TVTM), game consoles (for example, Xbox™ and PlayStation™), electronic dictionaries, electronic keys, camcorders, and el ectronic picture frames.

According to some embodiments of the present disclosure, an electronic device may include at least one of various medical devices supporting call forwarding s ervice (for example, various portable measurement devices (for example, glucometers, heart rate meters, blood pressure meters, temperature meters, etc.), magnetic resonance angiography (MRA) devices, magnetic resonance imaging (MRI) devices, computed to mography (CT) devices, medical imaging devices, ultrasonic devices, etc.), navigation devices, global positioning system (GPS) receivers, event data recorders (EDRs), flight data recorders (FDRs), vehicle infotainment devices, marine electronic equipment (for example, marine navigation systems, gyro compasses, etc.), avionics, security equipme nt, vehicle head units, industrial or household robots, financial institutions' automatic te ller's machines (ATMs), or stores' point of sales (POS) or internet of things (for examp le, bulbs, various sensors, electric or gas meters, sprinkler systems, fire alarms, thermos tats, street lights, toasters, exercise equipment, hot water tanks, heaters, boilers, etc.).

In various embodiments of the present disclosure, an electronic device m ay include at least one of part of furniture or buildings/structures supporting call forwar ding service, electronic boards, electronic signature receiving devices, projectors, and v arious measuring instruments (for example, water, electricity, gas, or radio signal meas uring instruments). An electronic device according to various embodiments of the prese nt disclosure may be one of the above-mentioned various devices or a combination ther eof. Additionally, an electronic device according to an embodiment of the present discl osure may be a flexible electronic device. Additionally, an electronic device according t o an embodiment of the present disclosure is not limited to the above-mentioned device s and may include a new kind of an electronic device according to the technology devel opment.

Hereinafter, an electronic device according to various embodiments of t he present disclosure will be described in more detail with reference to the accompanyi ng drawings. The term "user" in this disclosure may refer to a person using an electroni c device or a device using an electronic device (for example, an artificial intelligent ele ctronic device).

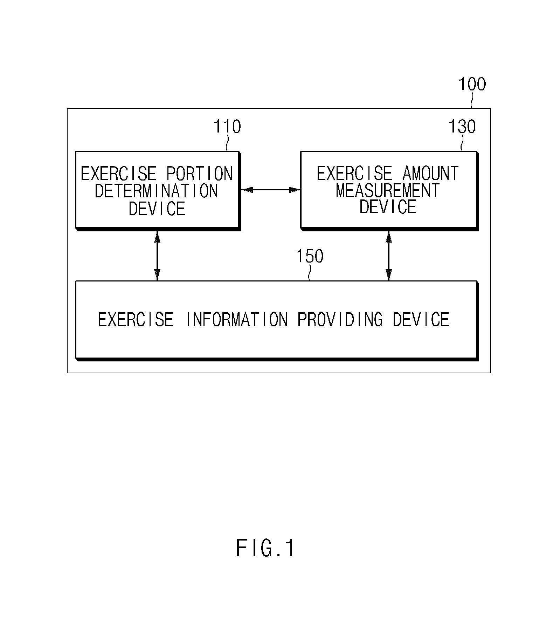

FIG. 1 is a view illustrating an exercise information providing system ac cording to various embodiments of the present disclosure.

Referring to FIG. 1, an exercise information providing system 100 accor ding to various embodiments of the present disclosure may include an exercise portion determination device 110, an exercise amount measurement device 130, and an exercis e information providing device 150.

The exercise portion determination device 110 may perform a function f or determining a body portion that is actually used during a specific exercise, for examp le, an exercise portion (or the type of a muscle or a muscle group). According to an em bodiment of the present disclosure, the exercise portion determination device 110 may determine an exercise portion on the basis of at least one of specified information indic ating at least expected a part of a muscle group affected by an exercise performed by a user, sensor information, and user input information. For example, the exercise portion determination device 110 may determine an exercise portion on the basis of at least one of exercise portion information preloaded to an exercise device, sensor information rec eived from a sensor module included in an exercise device (or an exercise auxiliary dev ice, such as, for example, a wearable exercise device), and information inputted by a us er.

According to various embodiments of the present disclosure, the exercis e portion determination device 110 may be a health device design to exercise a specifie d exercise portion or an electronic device included in the exercise device. For example, the exercise portion determination device 110 may be an exercise device equipped in a fitness center, for example, a fly pec deck machine. In this case, an exercise portion exe rcisable by an exercise device may be a chest portion (or a pectoralis major portion) an d a corresponding exercise device may have a chest portion (or the pectoralis major por tion) as specified exercise portion information.

According to various embodiments of the present disclosure, the exercis e portion determination device 110 may be an exercise device or an exercise auxiliary d evice, which includes a sensor module, or an electronic device included in an exercise d evice or an exercise auxiliary device. For example, the exercise portion determination d evice 110 may be a dumbbell including a gyro sensor or an acceleration sensor. In this case, the exercise portion determination device 110 may determine an exercise portion on the basis of a measurement value (for example, sensor information) of the sensor cor responding to a position change of a dumbbell, which occurs when a user exercise by u sing the dumbbell. For example, when a user perform an alternated dumbbell curl exerc ise by using a dumbbell, the exercise portion determination device 110 may determine an arm portion (or a biceps portion) as exercise portion information on the basis of a m easurement value of the sensor. According to an embodiment of the present disclosure, in order to determine an exercise portion on the basis of sensor information, the exercis e portion determination device 110 may collect various patterns of sensor a measureme nt value according to an operation of a typical exercise method from a storage module i ncluded in the exercise portion determination device 110 or an external electronic devic e connected to the exercise portion determination device 110 through wired/wireless co mmunication.

According to various embodiments of the present disclosure, the exercis e portion determination device 110 may be connected to an exercise device or an exerci se auxiliary device, which includes a sensor module, through wired/wireless communic ation. For example, the exercise portion determination device 110 may be a smartphone connected to a dumbbell including a gyro sensor or an acceleration sensor through Blu etooth. In this case, the exercise portion determination device 110 may determine an ex ercise portion by receiving sensor information from a dumbbell connected through Blue tooth.

According to various embodiments of the present disclosure, the exercis e portion determination device 110 may be an electronic device including an input devi ce. For example, the exercise portion determination device 110 may be a smartphone in cluding a touch panel. In this case, the exercise portion determination device 110 may d etermine a corresponding exercise portion by receiving the type of an exercise that a us er is to perform or the name of an exercise from the user. For example, when a user inp uts a pushup exercise, the exercise portion determination device 110 may determine a c hest portion (or a pectoralis major portion) as an exercise portion on the basis of user in put information. According to an embodiment of the present disclosure, the exercise po rtion determination device 110 may collect information on an exercise portion correspo nding to various exercises from a storage module included in the exercise portion deter mination device 110 or an external electronic device connected to the exercise portion d etermination device 110 through wired/wireless communication.

According to various embodiments of the present disclosure, the exercis e portion determination device 110 may determine an exercise portion on the basis of a plurality of information among specified information, sensor information, and user inpu t information. For example, when a user swims while wearing a glove having a sensor a ttached, the exercise portion determination device 110 may receive information (for exa mple, information on an exercise type (for example, swimming) from an arbitrary electr onic device installed at an entrance or a near location of a swimming pool or an arbitrar y electronic device installed at the glove. Additionally, the exercise portion determinati on device 110 may receive sensor information corresponding to a user's movement (for example, an exercise motion) through a sensor installed at the swimming pool or a sens or installed at the glove. In this case, the exercise portion determination device 110 may include patterns for a corresponding exercise type, for example, swimming, in an analy sis target among patterns of sensor measurement values according to various exercises on the basis of specified information and may determine an exercise motion that a user performs, for example, a swimming style, among the patterns on the basis of sensor inf ormation. Additionally, the exercise portion determination device 110 may determine a n exercise portion corresponding to a swimming style.

According to various embodiments of the present disclosure, the exercis e portion determination device 110 may receive exercise type related information from an electronic device installed at an exercise place (for example, a swimming pool), an e lectronic device disposed at an exercise device, or an electronic device disposed at an e xercise auxiliary device. Alternatively, the exercise portion determination device 110 m ay determine an exercise portion according to a specified exercise type or pattern throu gh a pattern analysis of sensor information measured by a sensor included in an exercis e place, an exercise device, or an exercise auxiliary device.

The exercise amount measurement device 130 may perform a function f or measuring an exercise amount performed during a specific exercise. According to an embodiment of the present disclosure, the exercise amount measurement device 130 m ay measure an exercise amount on the basis of sensor information received from a sens or module included in an exercise place, an exercise device or and an exercise auxiliary device. Alternatively, the exercise amount measurement device 130 may be configured in a form of being included in an exercise place, an exercise device or and an exercise auxiliary device, as including a sensor module for measuring an exercise amount, or the exercise amount measurement device 130 itself may be an exercise place, an exercise d evice or and an exercise auxiliary device.

In relation to this, the exercise amount measurement device 130 may col lect user's body information in order to measure a more accurate exercise amount. For e xample, the exercise amount measurement device 130 may collect information such as t he user's age, sex, weight, or height. According to an embodiment of the present disclos ure, the exercise amount measurement device 130 may collect user's body information f rom a user through an input device or from an external electronic device connected thro ugh wired/wireless communication. Alternatively, the exercise amount measurement de vice 130 may measure user's body information through a sensor module. For example, t he user's weight may be measured through a pressure sensor attached to sports shoes. A lternatively, the exercise amount measurement device 130 may additionally measure us er's body information such as the user's blood pressure, heart rate, muscle volume, body fat, or body mass index through a sensor module.

According to various embodiments of the present disclosure, the exercis e amount measurement device 130 may measure an exercise amount on the basis of use r's body information and a physical amount corresponding to sensor information receiv ed from a sensor module, for example, pressure, distance, time, speed, or acceleration. Additionally, the exercise amount measurement device 130 may measure calories cons umed during a specific exercise on the basis of a measured exercise amount or received sensor information. According to an embodiment of the present disclosure, in relation t o an exercise amount measurement, the sensor module may include a pressure sensor, o r an acceleration sensor. According to an embodiment of the present disclosure, in relati on to a function for measuring additional information such as blood pressure and heart r ate, the sensor module may include a heart rate sensor or a biometric sensor.

The exercise information providing device 150 may perform a function f or receiving information from the exercise portion determination device 110 and the ex ercise amount measurement device 130 and providing exercise information to a user. A ccording to an embodiment of the present disclosure, the exercise information providin g device 150 may provide to a user an exercise amount (for example, a muscle specific exercise amount) of an exercise portion a user actually uses during a specific exercise t hrough an output device on the basis of an exercise portion determined through the exer cise portion determination device 110 and an exercise amount measured through the ex ercise amount measurement device 130.

According to various embodiments of the present disclosure, the exercis e information providing device 150 may provide user's body information or exercise de tail information to a user. For example, when a user performs a specific exercise, as soo n as performing an exercise or terminating an exercise, the exercise information providi ng device 150 may output to an output device (for example, a speaker, an earphone, or a display) the information corresponding to user's body information (for example, the u ser's age, sex, weight, height, blood pressure, heart rate, muscle volume, body fat, or bo dy mass index) or exercise detail information (for example, an exercise portion corresp onding to a performed exercise, an exercise amount, an exercise portion specific exerci se amount, an exercise type, an exercise name, an exercise intensity, an exercise freque ncy, an exercise time, a calorie consumption amount, an exercise portion specific exerci se amount target value, or an exercise portion specific exercise amount remaining value ).

Although it is described above that the exercise information providing d evice 150 has a form configured separated from the exercise portion determination devi ce 110 and the exercise amount measurement device 130, according to an embodiment of the present disclosure, the exercise information providing device 150 may be configu red in a form of being included in the exercise portion determination device 110 or the exercise amount measurement device 130. Additionally, the exercise information provi ding system 100 may be configured in a form of including a plurality of at least one of t he components (for example, the exercise portion determination device 110, the exercis e amount measurement device 130, and the exercise information providing device 150.

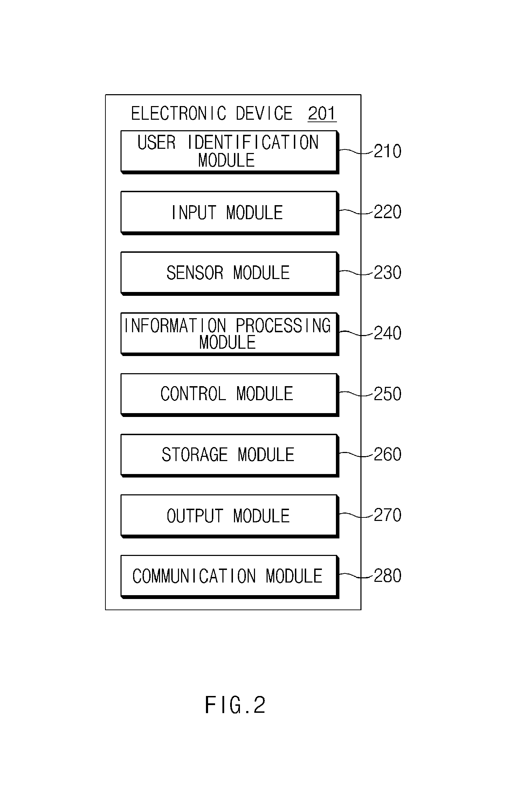

FIG. 2 is a view illustrating an electronic device in an exercise informati on providing system according to various embodiments of the present disclosure. An el ectronic device 201 may have the same or similar configuration to the exercise portion determination device 110, the exercise amount measurement device 130, and the exerci se information providing device 150, which are included in the exercise information pr oviding system 100 shown in FIG. 1.

Referring to FIG. 2, the electronic device 201 according to various embo diments of the present disclosure may include a user identification module 210, an inpu t module 220, a sensor module 230, an information processing module 240, a control m odule 250, a storage module 260, an output module 270, and a communication module 280. According to various embodiments of the present disclosure, the electronic device 201 may omit at least one of components or may additionally include another compone nt according to a function (for example, an exercise portion determination function, an exercise amount measurement function, or an exercise information providing function) of the electronic device 201.

When a user directly uses the electronic device 201 or a function include d in the electronic device 201, the user identification module 210 may perform a functi on for identifying a user. According to an embodiment of the present disclosure, the use r identification module 210 may identify a user on the basis of user identification infor mation (for example, a user name, a user identification number, or a user code) inputted from a user through the input module 220, biometric information (for example, a user f ingerprint, and so on) measured through the sensor module 230, or user identification in formation received from an external electronic device through the communication mod ule 280. According to an embodiment of the present disclosure, the user identification module 210 may process to store user identification information corresponding to the id entified user through the storage module 260.

The input module 220 may perform a function for receiving an input fro m a user. According to an embodiment of the present disclosure, the input module 220 may receive user identification information, user's body information, or the type or nam e of an exercise that a user is to perform, from a user. Additionally, the input module 22 0 may receive a user's input in relation to an exercise service that an exercise informatio n providing system (for example, the exercise information providing system 100 of FIG . 1) provides. The exercise service may be an exercise based health management servic e that comprehensively provides the type, name, intensity, frequency, time, or order of an exercise that a user performs.

The sensor module 230 may perform a function for measuring a physical amount or detecting an operating state of the electronic device 201. The sensor module 230 may include a pressure sensor, a gyro sensor, an acceleration sensor, a heart rate se nsor, a biometric sensor, an iris sensor, a fingerprint sensor, or a proximity sensor. Acc ording to an embodiment of the present disclosure, the sensor module 230 may detect w hether there is an approaching object or an object existing at a close location through a proximity sensor or may measure biometric information for identifying a user through a n iris sensor or a fingerprint sensor. Additionally, the sensor module 230 may measure a physical amount for determining an exercise portion through a gyro sensor or an accel eration sensor or may measure a physical amount for measuring an exercise amount thr ough a pressure sensor or an acceleration sensor. Additionally, the sensor module 230 may measure biometric information such as blood pressure and heart rate through a hea rt rate sensor or a biometric sensor.

The information processing module 240 may process to perform a functi on included in the electronic device 201 on the basis of user identification information i dentified through the user identification module 210, user input information received th rough the input module 220, sensor information (for example, a physical amount) meas ured through the sensor module 230. For example, the information processing module 2 40 may perform an exercise portion determination function, an exercise amount measur ement function, or an exercise information providing function on the basis of the user i dentification information, the user input information, or the sensor information.

According to various embodiments of the present disclosure, when the el ectronic device 201 is identical or similar to an exercise portion determination device (f or example, the exercise portion determination device 110 of FIG. 1), the information p rocessing module 240 may perform a function for determining an exercise portion on th e basis of at least one of the sensor information and the user input information. Additio nally, when the electronic device 201 is identical or similar to an exercise amount meas urement device (for example, the exercise amount measurement device 130 of FIG. 1), the information processing module 240 may perform a function for measuring an exerci se amount on the basis of at least one of the user identification information and the sens or information. Additionally, when the electronic device 201 is identical or similar to a n exercise information providing device (for example, the exercise information providi ng device 150 of FIG. 1), the information processing module 240 may perform a functi on for providing exercise information on the basis of at least one of the user input infor mation and the sensor information.

The control module 250 may execute calculation or data processing for c ontrol and/or communication of at least one another component included in the electron ic device 201. For example, the control module 250 may be a processor including one o r more of a central processing unit (CPU), an Application Processor (AP), and a comm unication processor (CP). According to various embodiments of the present disclosure, the control module 250 may be configured in a form of including the information proce ssing module 240 or may be configured in a form of being separated from the informati on processing module 240 so that it may perform calculation or data processing for con trol and/or communication in order to execute a function of the information processing module 240.

The storage module 260 may store instructions or data relating to at least one another component included in the electronic device 201. For example, the storage module 260 may store user identification information identified through the user identi fication module 210. Additionally, the storage module 260 may store information relati ng to a function performance of the information processing module 240 or information processed through the information processing module 240. According to an embodimen t of the present disclosure, when the information processing module 240 performs a fun ction for determining an exercise portion, the storage module 260 may store various pat terns of a sensor measurement value according to an operation of typical exercise meth od or an exercise portion determined based on the patterns. Additionally, when the info rmation processing module 240 performs a function for measuring an exercise amount, the storage module 260 may store a measured exercise amount.

According to various embodiments of the present disclosure, the storage module 260 may store an exercise service application in relation to an exercise service t hat an exercise information providing system (for example, the exercise information pr oviding system 100 of FIG. 1) provides. The exercise service application may be an ap plication for providing a user's health management service on the basis of an exercise, f or example, a function for recommending an exercise appropriate for a user according t o user's body information or user input information, providing information on an exerci se that a user performs, or designating and providing the order of exercises that a user is to perform. According to an embodiment of the present disclosure, the exercise service application may provide information such as the type, effect, method, intensity, or freq uency of an exercise appropriate for a user to a user according to an exercise order. Add itionally, the exercise service application may provide detail information of an exercise that a user performs, for example, the intensity, time, frequency, amount, or consumed calories of an exercise, to a user.

The output module 270 may perform a function for outputting informati on relating to a function performance of the electronic device 201. The output module 2 70 may include at least one of a display and an audio output device. According to an e mbodiment of the present disclosure, the output module 270 may output user's body inf ormation or exercise detail information. For example, the output module 270 may displ ay an image object such as a graph representing a muscle specific exercise amount used during a specific exercise on a display. Additionally, the output module 270 may outpu t voice information corresponding to an exercise frequency through a speaker or an ear phone.

The communication module 280 may set communication between electr onic devices (the exercise portion determination device 110, the exercise amount measu rement device 130, and the exercise information providing device 150) included in an e xercise information providing system (for example, the exercise information providing system 100 of FIG. 1) or may set communication with an external electronic device. Th e communication module 280 may be connected to a network through wired communic ation or wireless communication. For example, an exercise information providing devic e may be connected to a network through wired communication or wireless communica tion on the basis of the communication module 280 to communicate with an exercise p ortion determination device or an exercise amount measurement device. According to a n embodiment of the present disclosure, the communication module 280 may set comm unication for an IoT based exercise information providing system. The IoT based exerci se information providing system will be described with an embodiment below.

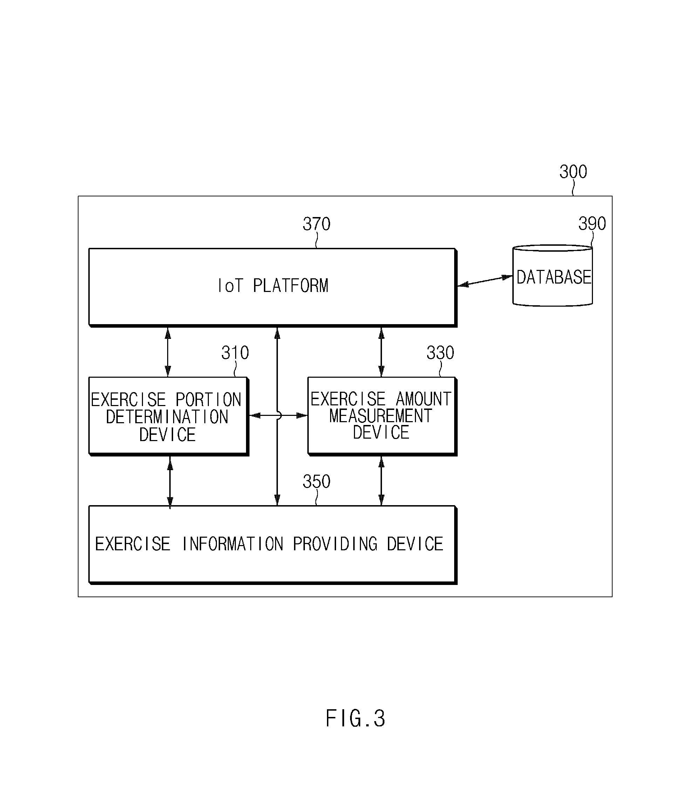

FIG. 3 is a view illustrating an IoT based exercise information providing system according to various embodiments of the present disclosure.

Referring to FIG. 3, an IoT exercise information providing system 300 a ccording to various embodiments of the present disclosure may include an exercise port ion determination device 310, an exercise amount measurement device 330, an exercise information providing device 350, an IoT platform 370, and a database 390. In the des cription below, content identical, similar, or corresponding to the above-mentioned cont ent may be omitted below.

Electronic devices included in the exercise information providing syste m 300 may be connected to each other through wired/wireless communication on the b asis of communication modules of the electronic devices. In this case, the IoT platform 370 may perform a function for managing and integrating electronic devices included i n the exercise information providing system 300.

According to various embodiments of the present disclosure, the IoT pla tform 370 may activate or deactivate electronic devices included in the exercise inform ation providing system 300 without a user's explicit instruction. According to an embod iment of the present disclosure, when a user directly uses the electronic devices, approa ches the electronic devices, or uses a function included in the electronic devices, the Io T platform 370 may activate corresponding electronic devices. For example, when a us er approaches or uses an exercise device included in the electronic devices, the IoT plat form 370 may activate the electronic devices to induce a function performance of the el ectronic devices. In relation to this, the IoT platform 370 may obtain the locations of us ers and the electronic devices on the basis of location based service (LBS). The LBS m ay be a system for providing various services to a user on the basis of location informat ion obtained through mobile communication network or global positioning system (GP S).

According to various embodiments of the present disclosure, electronic devices included in the exercise information providing system 300 may identify the app roach of a user on the basis of a proximity sensor included in the electronic devices. In t his case, in correspondence to a user's approach, the electronic devices may activate a c orresponding module for performing a function such as user identification or may deliv er a signal (for example, an event) corresponding to the user's approach to the IoT platf orm 370. According to an embodiment of the present disclosure, when a user directly u ses the electronic devices, approaches the electronic devices, or uses a function include d in the electronic devices, the IoT platform 370 may activate corresponding electronic devices.

According to various embodiments of the present disclosure, when a use r stop using the electronic devices, is away from the electronic devices, or terminates a function included in the electronic devices, the IoT platform 370 may deactivate corres ponding electronic devices.

According to various embodiments of the present disclosure, the IoT pla tform 370 may collect information from electronic devices included in the exercise info rmation providing system 300 and then, may integrate, determine, or control user specif ic information. According to an embodiment of the present disclosure, the IoT platform 370 may be a server for performing an exercise service function for a plurality of users . In this case, the IoT platform 370 may collect determined or measured information fro m the exercise portion determination device 310 and the exercise amount measurement device 330 and may integrate and manage information on the basis of user identificatio n information.

Additionally, the IoT platform 370 may determine an exercise appropria te for a user on the basis of user's body information or a preference for a specific exerci se, which is included in user specific integrated information. The preference for a specif ic exercise may be determined on the basis of information such as the number of execut ions, intensity, or frequency for a specific exercise. For example, when the number of e xecutions, the intensity, or the frequency for a specific exercise is relatively higher in c omparison to other exercises provided through exercise service, the IoT internet platfor m 370 may determine that a user's preference for a corresponding exercise is high. In t his case, the IoT platform 370 may determine an exercise portion according to a highly-preferred specific exercise and then may provide exercises for exercising a correspondi ng portion to a user.

According to various embodiments of the present disclosure, the IoT pla tform 370 may perform a control to store a user specific integrated information in a stor age module included in the IoT platform 370 or an external storage device (for example , the database 390). In relation to this, the user specific integrated information may be i nformation where user's body information, exercise history information, exercise detail information, and a preference for specific exercise are integrated on the basis of user id entification information.

According to various embodiments of the present disclosure, the IoT pla tform 370 may provide information utilized for function performance to electronic devi ces included in the exercise information providing system 300. According to an embodi ment of the present disclosure, in relation to specific exercise service provided to each user, the IoT platform 370 may deliver an exercise appropriate for a user to the exercise information providing device 350 according to the order of exercises to be performed a nd provide it to a user. In relation to this, the IoT platform 370 may use a user specific i ntegrated information stored in a storage module included in the IoT platform 370 or an external storage device (for example, the database 390). Additionally, the IoT platform 370 may deliver to the exercise portion determination device 310 the information on a n exercise place, an exercise device, or an exercise auxiliary device stored in the storag e module or the external storage device.

The database 390 may store information for performing functions of ele ctronic devices included in the exercise information providing system 300. According t o various embodiments of the present disclosure, the database 390 may include user's b ody information, or user's exercise history information in user specific integrated infor mation and store it. Additionally, the database 390 may store information on an exercis e place, an exercise device, or an exercise auxiliary device.

According to various embodiments of the present disclosure, the exercis e information providing device may include an information processing module configur ed to determine exercise information corresponding to an exercise portion specific exer cise amount on the basis of at least exercise portion information received from an exerc ise portion determination device and exercise amount information received from an exe rcise amount measurement device; an output module configured to output the exercise i nformation; and a communication module configured to provide an communication inte rface between the exercise portion determination device and the exercise amount measu rement device.

According to various embodiments of the present disclosure, the exercis e information providing device may be the exercise portion determination device or the exercise amount measurement device.

According to various embodiments of the present disclosure, the exercis e portion determination device may determine the exercise portion information on the b asis of at least one of user input information, specified information, information receive d through at least one sensor included in the exercise portion determination device, and information received through at least one sensor included in the exercise amount measu rement device.

According to various embodiments of the present disclosure, the specifie d information may include exercise portion information corresponding to at least one of exercise place information, exercise device information, and exercise auxiliary device i nformation, and is stored in at least one of a storage module included in the exercise po rtion determination device and an external storage device connected through wired/wire less communication.

According to various embodiments of the present disclosure, at least one of the information received through at least one sensor included in the exercise portion determination device and the information received through at least one sensor included in the exercise amount measurement device corresponds to pattern information accordi ng to an operation of a specified exercise method, and the pattern information may be st ored in at least one of a storage module included in the exercise portion determination d evice and an external storage device connected through wired/wireless communication.

According to various embodiments of the present disclosure, the exercis e amount measurement device may measure the exercise amount information on the ba sis of at least one of the information received through at least one sensor included in the exercise portion determination device and the information received through at least on e sensor included in the exercise amount measurement device.

According to various embodiments of the present disclosure, the exercis e information may include at least one of user's body information, exercise detail infor mation, and exercise list information; the user's body information may include at least one of user's age, sex, weight, height, blood pressure, heart rate, muscle volume, body f at, and body mass index; the exercise detail information may include at least one of the exercise portion corresponding to an exercise performed, the exercise amount, an exerci se portion specific exercise amount determined based on the exercise portion and the ex ercise amount, an exercise type, an exercise name, an exercise intensity, an exercise fre quency, an exercise time, calorie consumption amount measured on the basis of the use r's body information and the exercise amount, an exercise portion specific exercise amo unt target value, and an exercise portion specific exercise amount remaining value; and the exercise list information may include at least one of an exercise type, an exercise na me, an exercise intensity, an exercise frequency, an exercise time, and an exercise order , each of which corresponds to an exercise to be performed.

According to various embodiments of the present disclosure, the exercis e portion determination device, the exercise amount measurement device, and the exerc ise information providing device may be Internet of Things (IoT) devices connected to an IoT platform through wired/wireless communication; and the IoT platform may gene rate user specific integrated information identified by each user on the basis of the exer cise portion information received from the exercise portion determination device, the e xercise amount information received from the exercise amount measurement device, an d the exercise information received from the electronic device and store the generated u ser specific integrated information.

According to various embodiments of the present disclosure, the IoT pla tform may be connected to an external storage device storing at least one of exercise pl ace information, exercise device information, exercise auxiliary device information, an d the user specific integrated information, through wired/wireless communication.

According to various embodiments of the present disclosure, the output module may output at least one of voice information corresponding to the exercise infor mation and an object including a text or image corresponding to the exercise informatio n.

According to embodiments described below, an exercise information pro viding method based on various combinations of an exercise portion determination devi ce and an exercise amount measurement device in an exercise information providing sy stem and IoT will be described.

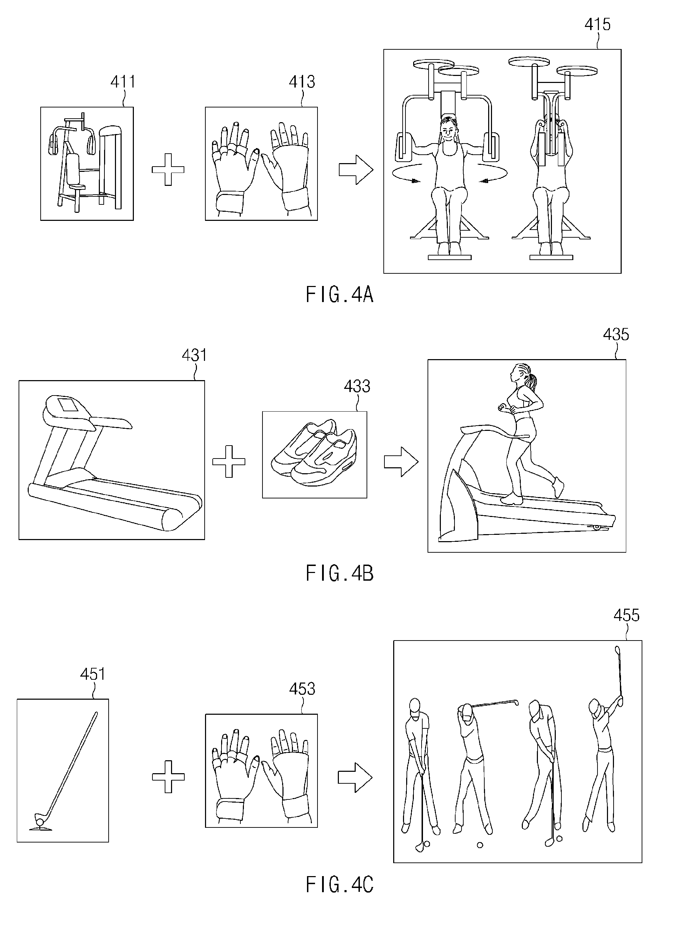

FIG. 4A is a first view illustrating an exercise portion determination and an exercise amount measurement on the basis of specified information according to var ious embodiments of the present disclosure, FIG. 4B is a second view illustrating an ex ercise portion determination and an exercise amount measurement on the basis of speci fied information according to various embodiments of the present disclosure, and FIG. 4C is a third view illustrating an exercise portion determination and an exercise amount measurement on the basis of specified information according to various embodiments of the present disclosure.

Referring to FIG. 4A, an exercise information providing system (for exa mple, the exercise information providing system 100 of FIG. 1) according to various e mbodiments of the present disclosure may include an exercise portion determination de vice 411 and an exercise amount measurement device 413. According to an embodimen t of the present disclosure, the exercise portion determination device 411 may be an "ex ercise portion specified exercise device," or essentially an exercise machine. Additiona lly, the exercise amount measurement device 413 may be an exercise auxiliary device ( for example, a wearable exercise device) that includes a sensor module for tracking the exercise done on the exercise machine. For example, the exercise portion determination device 411 may be a fly pec deck machine specified for exercising a portion of the user 's body, which in this case would be a chest portion (or a pectoralis major portion) and the exercise amount measurement device 413 may be an exercise glove that includes a pressure sensor or an acceleration sensor for tracking execution of the exercise routine f or the chest. In this case, when a user wears an exercise glove and performs chest exerc ises depicted in 415 using the fly pec deck machine, an exercise information providing device (not shown) may provide information (such as a number of repetitions) related t o the chest to a user on the basis of information collected from one or both of the exerci se portion determination device 411 and the exercise amount measurement device 413. For example, an exercise information providing device may provide the qualitative and/ or quantitative exercise information regarding the left chest portion and the right chest portion to a user on a plurality of bases, such as, for example, pressure, moving distanc e, moving time, or moving speed, which corresponds to sensor information generated b y the exercise glove worn on the left hand and/or right hand.

According to various embodiments of the present disclosure, as shown i n FIG. 4B, the exercise portion determination device 431 may be a treadmill for exercis ing at least legs (or a biceps femoris portion), quads, and calves. Additionally, the exerc ise amount measurement device 433 may be integrated or implemented as athletic shoe s which include a pressure sensor and/or an acceleration sensor. In this case, when a us er wears the athletic shoes and exercise 435 on the treadmill, an exercise information pr oviding device (not shown) may provide quantitative and/or qualitative information to a user, regarding the movements and other associated information, generated from the ex ercise portion determination device 431 and/or the exercise amount measurement devic e 433. For example, an exercise information providing device may separately or integra lly provide the qualitative and/or quantitative exercise information regarding each of th e user's left leg and the right leg to a user based on sensor information collected from e ach of the athletic shoes.

Additionally, an exercise information providing device may calculate a r atio of consumed calories corresponding to a muscle specific exercise amount of the bo th legs to total calories and may provide it to a user. For example, when total calories c onsumed while a user performs the running exercise 435 are 148 kcal and a pressure rat io of the left leg and the right leg, which are measured from a pressure sensor included i n the sports shoes, is 6.4:3.6, an exercise information providing device may output calo ries corresponding to an exercise amount of the left leg as 94.72 kcal and calories corre sponding to an exercise amount of the right leg as 53.28 kcal.

According to various embodiments of the present disclosure, as shown i n FIG. 4C, the exercise portion determination device 451 may be a golf club which may be used to exercise at least a back portion (or a latissimus dorsi portion), a stomach or "core" portion (or an abdominal muscle portion), and/or a leg portion (or a calves porti on). Additionally, the exercise amount measurement device 453 may be sports gloves ( e.g., golfing gloves) that includes a pressure sensor or an acceleration sensor. In this cas e, when a user wears exercise gloves and performs a golf swing exercise 455, an exerci se information providing device (not shown) may provide muscle specific data based o n qualitative and quantitative information on the exercise to a user, collected from the e xercise portion determination device 451 and the exercise amount measurement device 453.

According to various embodiments of the present disclosure, the exercis e portion determination device 451 may determine a plurality of exercise portions, or m uscle groups benefited or otherwise affected by the performed exercise. Herein, the exe rcise portion determination device 451 may identify exercise ratio information perform ed by each exercise portion according to the exercise type. For example, when a user pe rforms the golf swing exercise 455 by using a golf club, the exercise portion determinat ion device 451 may determine that an exercise portions are latissimus dorsi, abdominal muscle, and or calves on the basis of specified information (for example, an exercise ty pe, which in this case may indicate golf swing information). Additionally, during the ex ecution of the golf swing exercise 455, the exercise portion determination device 451 m ay identify that an exercise portion specific performed exercise ratio is 30% latissimus dorsi, 40% abdominal muscle, and 30% calves. According to various embodiments of t he present disclosure, the exercise portion determination device 451 may identify that a n exercise portion specific performed exercise ratio using a data retrieved from a databa se(for example, the database 390) in which exercise portion specific performed exercise ratios/percentages are stored. In this case, if total consumption calories measured throu gh the exercise amount measurement device 453 by performing the golf swing exercise 455 are 129 kcal, an exercise information providing device may determine that latissim us dorsi of 38.7kcal, abdominal muscle of 51.6kcal, and calves of 38.7kcal as consume d calories corresponding to respective performed exercise ratios. According to various embodiments of the present disclosure, the exercise information providing device may determine that consumed calories corresponding to respective performed exercise ratio using a data retrieved from a database(for example, the database 390) in which consum ed calories corresponding to respective performed exercise are stored.

According to various embodiments of the present disclosure, an exercise information providing device may additionally provide information such as an exercise intensity, an exercise time, an exercise frequency, or an exercise type, which is benefic ial for a user, on the basis of user's exercise intensity, exercise time, exercise frequency , or each muscle specific exercise amount. Additionally, an exercise information provid ing device may provide a correct exercise posture to a user by analyzing a user's exerci se posture on the basis of sensor information received from at least one of an exercise p ortion determination device and an exercise amount measurement device. According to an embodiment of the present disclosure, in relation to providing a correct exercise post ure, an exercise information providing device may collect patterns of sensor measurem ent values corresponding to a correct posture for various exercises from a storage modu le included in an exercise information providing device and an external electronic devic e connected to the exercise information providing device through wired/wireless comm unication.

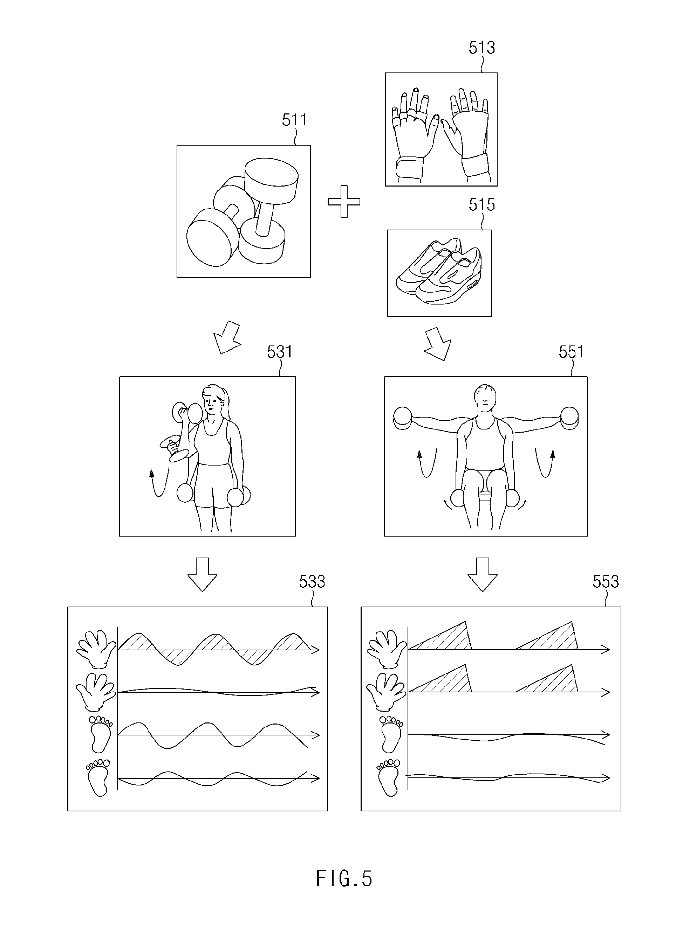

FIG. 5 is a view illustrating an exercise portion determination and an exe rcise amount measurement on the basis of sensor information according to various emb odiments of the present disclosure.

Referring to FIG. 5, an exercise information providing system (for exam ple, the exercise information providing system 100 of FIG. 1) according to various emb odiments of the present disclosure, and may include, as above, an exercise portion deter mination device 511, a first exercise amount measurement device 513, and a second ex ercise amount measurement device 515. According to an embodiment of the present dis closure, the exercise portion determination device 511 may include a sensor module. A dditionally, the first exercise amount measurement device 513 and the second exercise amount measurement device 515 may be exercise auxiliary devices (for example, a wea rable exercise device) that also include sensor modules. For example, the exercise porti on determination device 511 may be a dumbbell including a gyroscopic sensor and/or a n acceleration sensor. Additionally, the first exercise amount measurement device 513 may be sports gloves including a pressure sensor and/or an acceleration sensor, and the second exercise amount measurement device 515 may be sports shoes including a press ure sensor and/or an acceleration sensor.

According to various embodiments of the present disclosure, when a use r exercises with the dumbbells 511, they (e.g., the exercise portion determination devic e 511) may determine an exercise portion on the basis of sensor information measured t hrough a sensor module included in the exercise portion determination device 511 and a specific pattern of a sensor measurement value according to various exercises. For ex ample, the exercise portion determination device 511 may identify user's body informat ion (for example, a user's height) and a location relationship (for example, a distance b etween respective portions of a user's body (such as between an elbow, a shoulder and a dumbbell) between a user and a dumbbell on the basis of a sensor module (for exampl e, a proximity sensor) included in the exercise portion determination device 511. Additi onally, the exercise portion determination device 511 may determine a movement trajec tory of the dumbbell on the basis of a sensor value taking measurements during executi on of a specific exercise, and may identify the type of an exercise by using, for example , a combination of the detected location relationship and the movement trajectory. In thi s case, the exercise portion determination device 511 may determine an exercise portio n or a portion of the user's body being exercised, based on correspondence to the exerci se type.

According to various embodiments of the present disclosure, a user may wear IoT-enabled sports gloves and/or sports shoes and perform exercises such as alter nated dumbbell curls 531 or side lateral raises 551 using an IoT-enabled dumbbell. Dur ing performance of the alternated dumbbell curls 531, sensor modules included in the fi rst exercise amount measurement device 513 and the second exercise amount measure ment device 515 may measure sensor information, represented by the first graph 533. A n exercise information providing device (not shown) may measure a pressure ratio corr esponding to the left arm portion and the right arm portion on the basis of sensor infor mation corresponding to the first graph 533. Additionally, an exercise information prov iding device may identify the qualitative and quantitative exercise data of the left arm a nd the right arm by dividing the performed total exercise amount by the pressure ratio. I n this case, an exercise information providing device may provide this data, specific to a muscle used to the user based on the received/detected sensor information. According to an embodiment of the present disclosure, an exercise information providing device may also display or otherwise provide to a user the first graph 533, in the form of an im age, video or other visual media. Additionally, when an exercise performed by a user is the side lateral raise 551, sensor modules included in the first exercise amount measure ment device 513 and the second exercise amount measurement device 515 may measur e sensor information corresponding to a second graph 553.

According to various embodiments of the present disclosure, an exercise information providing device may provide a correct exercise posture by analyzing a us er's exercise posture on the basis of a muscle specific exercise amount of the sensor inf ormation. Additionally, an exercise information providing device may provide informat ion such as an exercise intensity, an exercise time, or an exercise frequency of a user.

According to various embodiments of the present disclosure, the exercis e portion determination device 511 may determine an exercise portion by analyzing a p hysical amount (for example, location, pressure, distance, time, speed, or acceleration) measured through sensor modules included in the first exercise amount measurement d evice 513 or the second exercise amount measurement device 515. Additionally, the fir st exercise amount measurement device 513 or the second exercise amount measureme nt device 515 may measure an exercise amount by analyzing a physical amount measur ed through a sensor module included in the exercise portion determination device 511. According to various embodiments of the present disclosure, the exercise portion deter mination function or the exercise amount measurement function may be performed bas ed on sensor modules of electronic devices included in an exercise information providi ng system through an exercise information providing device.

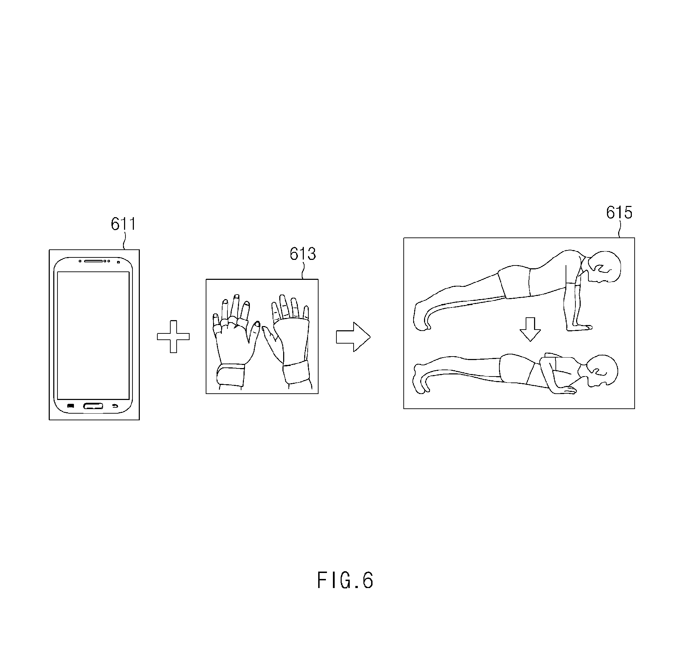

FIG. 6 is a view illustrating an exercise portion determination and an exe rcise amount measurement on the basis of user input information according to various e mbodiments of the present disclosure.

Referring to FIG. 6, an exercise information providing system (for exam ple, the exercise information providing system 100 of FIG. 1) according to various emb odiments of the present disclosure may include an exercise portion determination devic e 611 and an exercise amount measurement device 613. According to an embodiment o f the present disclosure, the exercise portion determination device 611 may be an electr onic device including an input device, such as a portable terminal or a smartphone. Add itionally, the exercise amount measurement device 613 may include a sensor module. F or example, the exercise portion determination device 611 may be a smartphone includi ng an input device. Additionally, the exercise amount measurement device 613 may be sports gloves including a pressure sensor.

According to various embodiments of the present disclosure, after desig nating a pushup exercise to the smartphone 611 and equipping the sports gloves 613, a user may perform a pushup exercise 615. In this case, the exercise portion determinatio n device 611 may detected and deliver an exercise portion (e.g., a muscle group) corres ponding to the pushup exercise to an exercise information providing device (not shown ). Additionally, the exercise amount measurement device 613 may deliver qualitative a nd quantities exercise information measured through a sensor module to the exercise in formation providing device. For example, the exercise portion determination device 61 1 may deliver an arm portion (or a biceps portion) as an exercise portion used during a pushup exercise to an exercise information providing device, and the exercise amount measurement device 613 may deliver sensor information corresponding to a physical a mount such as pressure measured through a sensor module included in sports gloves, or a number of detected repetitions, to an exercise information providing device.

In relation to this, the exercise portion determination device 611 may col lect information on an actually used exercise portion according to various exercise from a storage module included in the exercise portion determination device 611. Alternativ ely, the exercise portion determination device 611 may collect information on an actual ly used exercise portion according to various exercise from an external electronic devic e connected to the exercise portion determination device 611 via wire/wireless commun ication through a communication module. According to an embodiment of the present d isclosure, an exercise portion determination device may be configured in a form of bein g included in the exercise portion determination device 611.

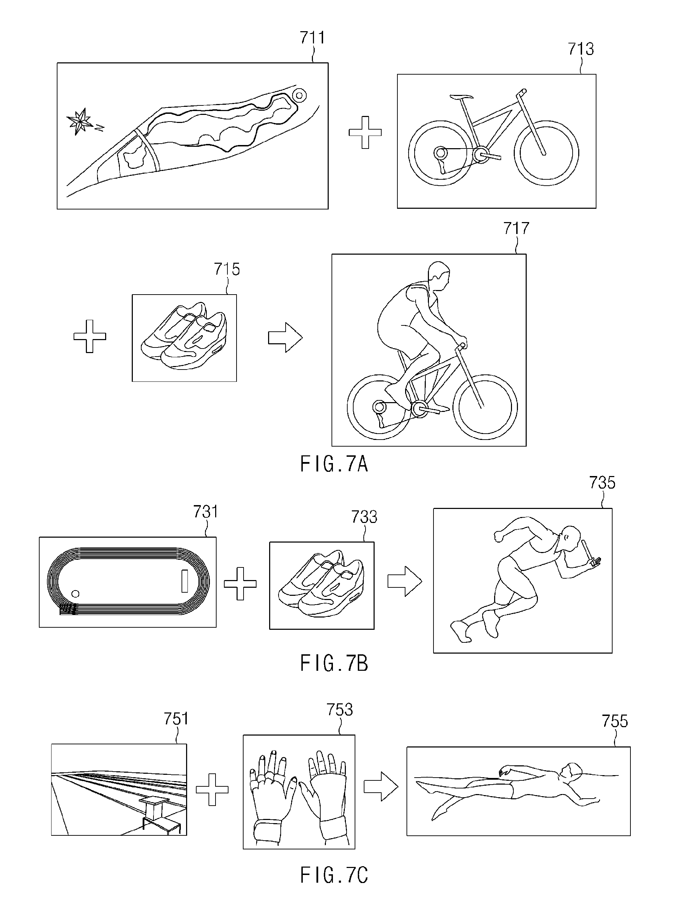

FIG. 7A is a first view illustrating an exercise portion determination and an exercise amount measurement on the basis of a plurality of information according to various embodiments of the present disclosure, FIG. 7B is a second view illustrating a n exercise portion determination and an exercise amount measurement on the basis of a plurality of information according to various embodiments of the present disclosure, an d FIG. 7C is a third view illustrating an exercise portion determination and an exercise amount measurement on the basis of a plurality of information according to various em bodiments of the present disclosure.

According to various embodiments of the present disclosure, as shown i n FIG. 7A, a user may exercise by riding a bicycle riding 717 in a park 711 using a bicy cle 713 and wearing sports shoes 715. According to an embodiment of the present discl osure, a user may attach a smartphone to the bicycle 713 or carry a smartphone, and an electronic device including an input/output device or a sensor module may be installed at a predetermined area of the bicycle 713. Additionally, a user may collect map inform ation on the park 711. The map information may be obtained from an arbitrary electron ic device installed at an entrance or a close location of the park 711 by using an electro nic device installed at a smartphone or the bicycle 713, or through an external electroni c device connected via network, such as a remote server. In this case, the collected map information on the park 711 may include such as distance, direction, altitude, or groun d type and an exercise information providing device may provide the information to a u ser.

According to various embodiments of the present disclosure, in relation t o the bicycle riding exercise 717, an exercise portion determination device may be an el ectronic device installed at a smartphone or the bicycle 713. Additionally, an exercise a mount measurement device may be an electronic device installed at the sports shoes 71 5 or the bicycle 713 including a sensor module. According to an embodiment of the pre sent disclosure, an exercise portion determination device may determine an exercise po rtion on the basis of user input information through an input device of a smartphone. Al ternatively, an exercise portion determination device may determine an exercise portion through an electronic device installed at the bicycle 713. In relation to this, an electroni c device installed at the bicycle 713 may include an input device, specify exercise porti on information, or include a sensor module. According to an embodiment of the present disclosure, an exercise amount measurement device may measure an exercise amount on the basis of a sensor module included in the sports shoes 715 or may measure an exe rcise amount on the basis of an electronic device installed at the bicycle 713. In relation to this, an electronic device installed at the bicycle 713 may include a sensor module or may be connected to a bicycle component (for example, a pedal) including a sensor mo dule.

According to various embodiments of the present disclosure, an exercise portion determination device or an exercise amount measurement device may determin e an exercise portion or measure an exercise amount by additionally using the collected map information of the park 711. An actually used exercise portion and an exercise am ount may vary according to the distance, direction, altitude, or ground type of the park 711. An exercise information providing device may include an object corresponding to an exercise portion or an exercise amount determined based on the map information of the park 711 in the map of the park 711 and provide it to a user.

According to various embodiments of the present disclosure, as shown i n FIG. 7B, a user may perform a running exercise 735 in a track 731 wearing sports sho es 733. According to an embodiment of the present disclosure, a user may perform the r unning exercise 735 while wearing a smart watch on a wrist. A user may collect inform ation on the track 731 from an arbitrary electronic device installed at an entrance or a cl ose location of the track 731 through a smart watch, or may collect information on the t rack 731 through an external electronic device connected via network. In this case, the collected information of the track 731 may include information such as a length or a gr ound type.

According to various embodiments of the present disclosure, in relation t o the running exercise 735, an exercise portion determination device may be an IoT-ena bled smart watch and an exercise amount measurement device may be IoT-enabled spo rts shoes 733 that include a sensor module. For example, an exercise portion determinat ion device may determine an exercise portion on the basis of user input information thr ough an input device of the smart watch. Additionally, an exercise portion determinatio n device may determine an exercise portion on the basis of information of the track 731 collected through a smart watch or a sensor module included in the sports shoes 733. F or example, an exercise portion determination device may determine that an exercise to be performed by a user is walking or running on the basis of the information of the trac k 731. In this case, an exercise portion determination device may include patterns whic h corresponding to walking or running among various sensor patterns for an analysis tar get. Additionally, an exercise portion determination device may determine that an exerc ise performed by a user is running through the comparison of sensor information measu red through a sensor module included in the sports shoes 733 and a stored sensor patter n.

According to various embodiments of the present disclosure, an exercise portion determination device may collect exercise portion information used according t o an exercise type from a storage module or an external electronic device connected thr ough a communication module. When a user performs the running exercise 735, an exe rcise portion determination device may determine exercise portion information accordi ng to the running exercise 735 on the basis of stored information or received informatio n and may deliver a corresponding exercise portion to an exercise information providin g device.

According to various embodiments of the present disclosure, an exercise amount measurement device may measure an exercise amount on the basis of a sensor module included in the sports shoes 733. For example, an exercise amount measuremen t device may measure an exercise amount on the basis of a measured or detected quantit y, being physical or otherwise (for example, pressure, moving distance, moving time, o r moving speed) and corresponding to sensor information measured through a pressure sensor or an acceleration sensor included in the sports shoes 733.

According to various embodiments of the present disclosure, an exercise amount measurement device may measure an exercise amount by additionally using at least one of information of the track 731 and sensor information collected through a sen sor module installed at the track 731. For example, an exercise amount may vary accor ding to a ground type. Additionally, when a sensor for recognizing a user is installed at the track 731, an exercise amount measurement device may measure an exercise amoun t on the basis of sensor information collected through the sensor, for example, an exerci se distance or a moving speed.

According to various embodiments of the present disclosure, as shown i n FIG. 7C, a user may perform a swimming exercise 755 in the swimming pool 751 wh ile wearing sports gloves 753. According to an embodiment of the present disclosure, a user may additionally wear a smart watch, wear swimming glasses, or carry a smartpho ne. In this case, a user may collect information on the swimming pool 751 from an arbit rary electronic device installed at an entrance or a close location of the swimming pool 751 through a smart watch, swimming glasses, or a smartphone, or may collect informa tion on the swimming pool 751 through an external electronic device connected via net work. In this case, the collected information of the swimming pool 751 may include inf ormation such as a length or a water depth.

According to various embodiments of the present disclosure, a smart wat ch, swimming glasses, or a smartphone may include the electronic device 201 of FIG. 2 , or may be an electronic device having the same or similar configuration to the electro nic device 201. For example, the swimming glasses may include a display device in an area (that is, an area for obtaining visibility (for example, a lens area) surrounded by a r im. Additionally, the swimming glasses may include a communication module in a cert ain portion such as a rim, a band, or a bridge. According to an embodiment of the prese nt disclosure, a smart watch, swimming glasses, or a smartphone may be used as an exe rcise portion determination device, an exercise amount measurement device, or an exer cise information proving device.

According to various embodiments of the present disclosure, in relation t o the swimming exercise 755, an exercise portion determination device may be a smart watch or a smartphone and an exercise amount measurement device may be the sports gloves 753 including a sensor module. For example, an exercise portion determination device may determine an exercise portion on the basis of user input information throug h an input device of a smart watch or a smartphone. Alternatively, an exercise portion d etermination device may determine an exercise portion on the basis of information of th e swimming pool 751 collected through the smart watch or the smartphone or a sensor module included in the exercise gloves 753. For example, an exercise portion determin ation device may determine that an exercise performed by a user is swimming on the ba sis of information of the swimming pool 751, determine a swimming style through a se nsor module included in the exercise gloves 753, and determine an exercise portion (e.g ., muscle group) corresponding to a corresponding swimming style. Additionally, an ex ercise amount measurement device may measure an exercise amount on the basis of a s ensor module included in the sports gloves 753.

According to various embodiments of the present disclosure, an exercise amount measurement device may measure an exercise amount by additionally using at least one of information of the swimming pool 751 and sensor information collected thr ough a sensor module installed at the swimming pool 751. Additionally, an exercise inf ormation providing device may provide exercise detail information such as an exercise portion, an exercise amount, and a muscle specific exercise amount to a user. For exam ple, an exercise information providing device may be swimming glasses. In this case, a n exercise information providing device may display information collected from an exe rcise portion determination device or an exercise amount measurement device during s wimming or information analyzed based on the collected information, in a predetermin ed area of a display included in swimming glasses.

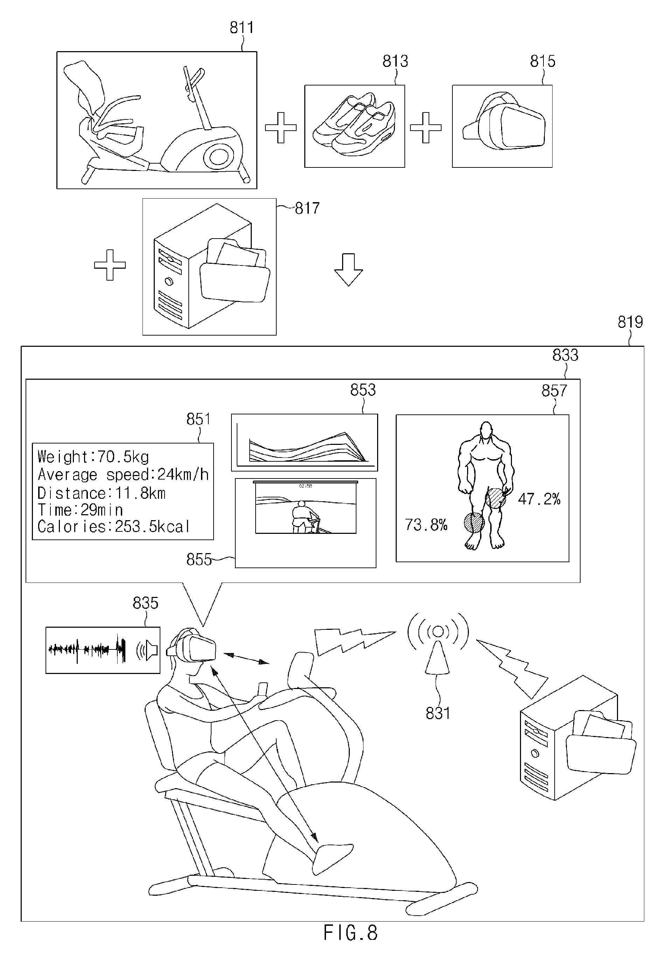

FIG. 8 is a view illustrating an IoT based exercise information providing system according to various embodiments of the present disclosure.

Referring to FIG. 8, an IoT based exercise information providing system (for example, the exercise information providing system 300 of FIG. 3) according to v arious embodiments of the present disclosure may include an exercise portion determin ation device 811, an exercise amount measurement device 813, an exercise information providing device 815, and an IoT platform 817. According to an embodiment of the pr esent disclosure, the exercise portion determination device 811 may be a fixed bicycle f or exercising a leg portion or a user's body (or a biceps femoris portion, a quads portion , and a calves portion) and the exercise amount measurement device 813 may be sports shoes including an attached pressure sensor. Additionally, the exercise information pro viding device 815 may be a head mount type display device (for example, a virtual reali ty or VR display) for implementing virtual reality, and the IoT platform 817 may be a s erver for storing user specific exercise history information, exercise device information, and/or exercise auxiliary device information.

According to various embodiments of the present disclosure, the exercis e portion determination device 811, the exercise amount measurement device 813, the e xercise information providing device 815, and the IoT platform 817 may be connected t o a network 831 through wired/wireless communication and therefore in intercommuni cation with one another. According to an embodiment of the present disclosure, when a user directly or indirectly uses electronic devices in an IoT-based exercise information providing system, the IoT platform 817 may activate the requisite electronic devices. A dditionally, the IoT platform 817 may collect, integrate, or analyze information obtaine d through operation and/or performance of each of the electronic devices, and similarly may provide information utilized for particular functions of each of the electronic devic es to the corresponding electronic devices.

According to an embodiment of the present disclosure, when a user perf orms an exercise 819 using the fixed bicycle, the IoT platform 817 may determine an e xercise appropriate for a user on the basis of user's exercise history information or user 's body information. Additionally, the IoT platform 817 may provide an exercise servic e to a user on the basis of the determined exercise. For example, the IoT platform 817 may display information such as an exercise type, an exercise time, an exercise intensit y, or an exercise frequency corresponding to a determined exercise on a display include d in the exercise information providing device 815, or may output voice information 83 5 through an audio output device. Additionally, the IoT platform 817 may perform a co ntrol to allow an exercise device (for example, a fixed bicycle) to automatically set the i ntensity of an exercise (for example, a bicycle gear or overall level of difficulty) on the basis of information corresponding to the determined exercise. According to various e mbodiments of the present disclosure, the IoT platform 817 may collect information cor responding to various VR environments from a storage module included in the IoT plat form 817 or an external electronic device connected through a communication module and may deliver the information to the exercise information providing device 815 for di splay. In this case, the IoT platform 817 or the exercise information providing device 8 15 may perform a control to deliver the intensity of an exercise corresponding to virtual reality information during exercise performance to an exercise device in real time in or der for automatic setting.

According to various embodiments of the present disclosure, the exercis e information providing device 815 may provide exercise detail information 833 or voi ce information 835 to a user. The exercise detail information 833 may be information c orresponding to an exercise status or an exercise result. This information may be provi ded to a user either as soon as an exercise is performed or as upon the exercise being de tected as terminated. Additionally, the voice information 835 may be voice information corresponding to exercise detail information. For example, the voice information 835 may include voice information corresponding to information such as an exercise type, a n exercise intensity, an exercise time, or an exercise frequency.

The exercise detail information 833 may include a text box 851 includin g user's body information, exercise distance, exercise time, or consumed total calories, a graph object 853 according to a time, an exercise screen 855 set through virtual realit y, or a muscle specific exercise amount image object 857. The text box 851 may be an object where information corresponding to user's body information, exercise status, or exercise result is expressed in a text format. The graph object 853 may be an object wh ere information corresponding to an exercise status or an exercise result is expressed in a graph format. For example, the graph object 853 may be a graph object corresponding to a total exercise amount or a muscle specific exercise amount determined or measure d as soon as an exercise starts or after a predetermined time. The exercise screen 855 m ay be a screen where information corresponding to various virtual realities is displayed using an object such as an image. Through the exercise screen 855, a user may receive an actually exercising feeling in various virtual realities. The image object 857 may be an object where a muscle specific exercise amount corresponding to an exercise status or an exercise result is displayed using a body's muscle image.

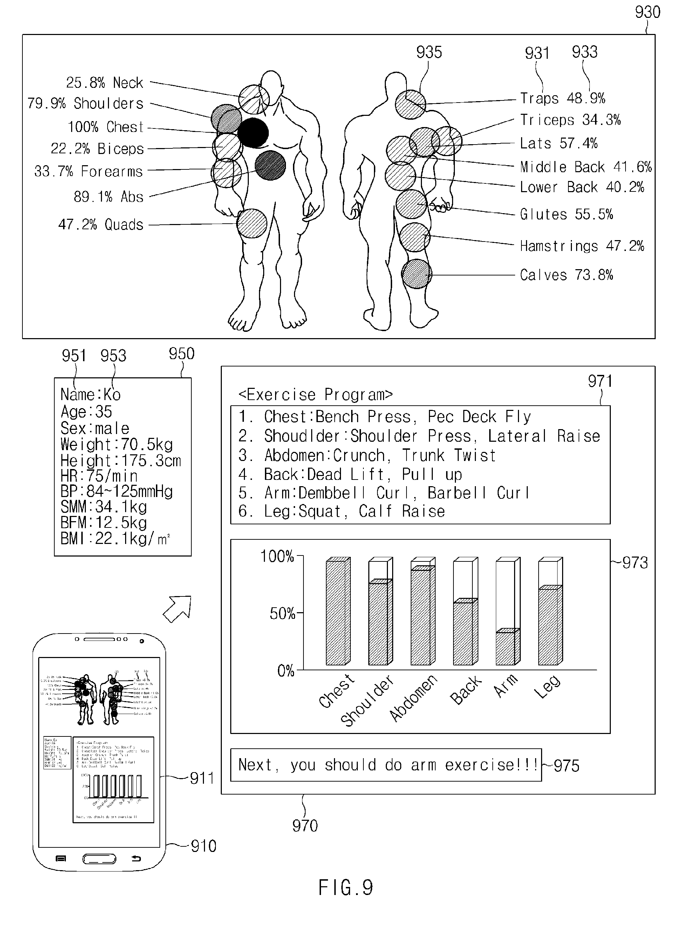

FIG. 9 is a view illustrating an output screen of an exercise information providing device 910 according to various embodiments of the present disclosure.

Referring to FIG. 9, an exercise information providing device 910 accor ding to various embodiments of the present disclosure may output a muscle specific exe rcise amount image 930, a text box 950 including user's body information, or an object 970 including exercise service information, to an output screen 911.

The muscle specific exercise amount image 930 may be an object where a muscle specific exercise amount corresponding to an exercise status or an exercise re sult is displayed using a body's muscle image. According to an embodiment of the pres ent disclosure, the muscle specific exercise amount image 930 may include a muscle na me 931, a muscle specific exercise amount 933, or a muscle location 935. The muscle n ame 931 may include a text corresponding to the name of an exercise portion specific muscle and may be disposed at a place adjacent to the muscle location 935 of the corres ponding muscle. The muscle specific exercise amount 933 may include information for displaying a muscle specific exercise amount performed by a user in a specified form. The muscle specific exercise amount 933, for example, may be displayed in percentage on the basis of a recommended exercise or may be displayed in consumed calories. The muscle specific exercise amount 933 may be located adjacent to a related muscle name 931 or a muscle location 935. The muscle location 935 may include a predetermined ar ea on the basis of a point where a related muscle is located or an adjacent point thereof and may be displayed as an object such an image. Additionally, the object correspondin g to the muscle location 935 may vary the color (for example, color, brightness, or satur ation) or transparency of the object on the basis of information corresponding to the mu scle specific exercise amount 933.

The text box 950 including user's body information may include inform ation such as the user's name, age, sex, weight, or height or additional information such as blood pressure, heart rate, muscle volume, body fat, or body mass index. The text bo x 950 including user's body information may include information 953 corresponding to an index 951 and the information 953 corresponding to the index 951 may be expresse d in a text format.

The object 970 including exercise service information may include infor mation for providing an exercise appropriate for a user. The object 970 including exerci se service information may include a text box 971 including a muscle specific exercise type, a muscle specific exercise amount graph object 973, or a notification message obj ect 975.

The text box 971 including a muscle specific exercise type may be an ob ject where the type of an exercise to be performed for the purpose to maximize a muscl e specific exercise is displayed. According to an embodiment of the present disclosure, when a specific area, for example, an area where an individual exercise (for example, a bench press, and so on) included in a list of muscles (for example, a chest, and so on) is displayed, is selected, the exercise information providing device 910 may display infor mation on an individual exercise corresponding to the selected area through a method s uch as pop-up or screen switching. For example, the exercise information providing de vice 910 may include the information on the individual exercise in a pop-up object in c orrespondence to the selection and may display it in a predetermined area of an output s creen 911. Alternatively, the exercise information providing device 910 may configure a screen including the information on the individual exercise and display it on the outpu t screen 911. According to various embodiments of the present disclosure, information on the type of an exercise included in the text box 971 including the type of a muscle sp ecific exercise or information on a related individual exercise may be information pre-s tored in a storage module included in the exercise information providing device 910 or information received from an external electronic device connected to the exercise infor mation providing device 910 through wired/wireless communication.

The muscle specific exercise amount graph object 973 may be an object for displaying a muscle specific exercise amount performed by a user according to an e xercise service provided to a user, as a graph. The muscle specific exercise amount gra ph object 973 may include information for displaying the degree of a muscle specific e xercise amount performed based on a muscle specific recommended exercise amount, i n a specified form. A muscle specific exercise amount included in the muscle specific e xercise amount graph object 973 may be displayed in percentage (%) Additionally, the muscle specific exercise amount graph object 973 may be may display an image object corresponding to a muscle specific exercise amount in an area corresponding to a relate d muscle.

In relation to an exercise service provided to a user, the notification mes sage object 975 may be an object such as a text or an image displaying information that the exercise information providing device 910 to a user. The notification message obje ct 975 may include information corresponding to an exercise type, an exercise order, an exercise intensity, or an exercise frequency, which is appropriate for a user. Alternativ ely, the notification message object 975 may include information corresponding to an e xercise frequency during a specific exercise, an exercise amount to reach a target value, a remaining value of an exercise amount to be performed. In the shown drawing, altho ugh it is described that the notification message object 975 is included in the object 970 including exercise service information, the notification message object 975 may be dis played in a pop-up form. Additionally, voice information corresponding to information included in the notification message object 975 may be outputted through a voice outpu t device.

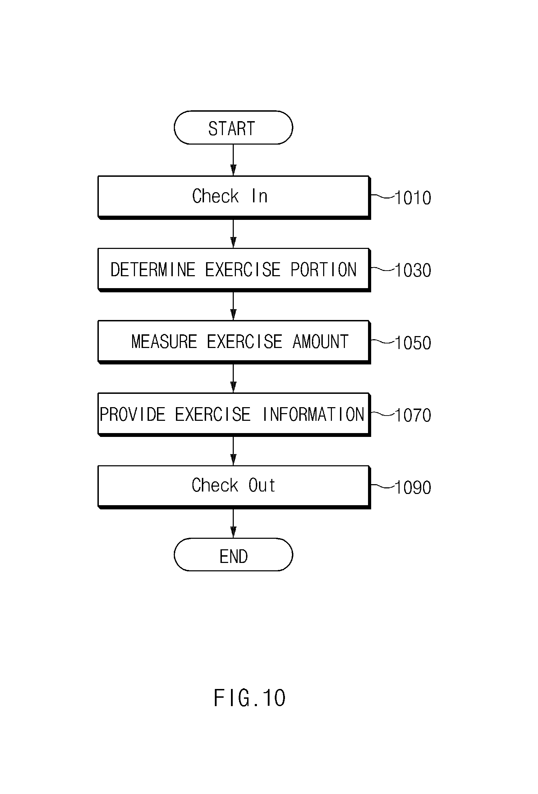

FIG. 10 is a view illustrating a method of operating an exercise informat ion providing system relating to an individual exercise according to various embodimen ts of the present disclosure.

Referring to FIG. 10, in relation to an exercise service provided from an exercise information providing system (for example, the exercise information providing system 100 of FIG. 1), according to an exercise information providing method an indiv idual exercise performed according to an exercise order included in exercise list inform ation, the exercise information providing system may perform a "check in" operation in operation 1010. According to an embodiment of the present disclosure, when a user us es a specified electronic device (for example, at least one of an exercise portion determi nation device (for example, the exercise portion determination device of FIG. 1), an exe rcise amount measurement device (for example, the exercise amount measurement devi ce of FIG. 1), and an exercise information providing device (for example, the exercise i nformation providing device 150 of FIG. 1) during a specific exercise, the electronic de vice may perform a function for identifying a user through a user identification module. For example, the electronic device may identify a user on the basis of user identificatio n information input from a user through an input module, biometric information measur ed through a sensor module, and user identification information received from an exter nal electronic device through a communication module. Additionally, the electronic de vice may process to store user identification information corresponding to the identified user through a storage module.

According to various embodiments of the present disclosure, in performi ng at least one of an exercise portion determination function, an exercise amount measu rement function, and an exercise information providing function, an exercise informatio n providing system may use user's body information collected from a storage module i ncluded in the electronic device or an external electronic device connected to the electr onic device through a communication module on the basis of the user's identification in formation identified through a check in operation.

In operation 1030, an exercise information providing system may perfor m a function for determining an exercise portion. According to an embodiment of the p resent disclosure, an exercise portion determination device included in an exercise infor mation providing system may perform a function for determining an exercise portion th at is actually used during a specific exercise on the basis of at least one of specified info rmation, sensor information, and user input information. In relation to this, an exercise portion determination device may collect exercise portion information specified to an e xercise place (such as a physical location), an exercise device (such as exercise equipm ent or installations), or an exercise auxiliary device (such as sensor-equipped gloves or equipment, or a portable terminal) from an arbitrary electronic device included in the e xercise place, the exercise device, or the exercise auxiliary device. According to an em bodiment of the present disclosure, an exercise portion determination device may be an arbitrary electronic device included in the exercise place, the exercise device, or the exe rcise auxiliary device. According to various embodiments of the present disclosure, an exercise portion determination device may determine an exercise portion on the basis o f sensor information collected from a sensor module included in the exercise portion de termination device or an arbitrary electronic device connected to the exercise portion de termination device through a communication module. Additionally, an exercise portion determination device may determine an exercise portion on the basis of user input infor mation collected from an input module included in the exercise portion determination d evice or an arbitrary electronic device connected to the exercise portion determination d evice through a communication module.

In operation 1050, an exercise information providing system may perfor m a function for measuring an exercise amount. According to an embodiment of the pre sent disclosure, an exercise amount measurement device in an exercise information pro viding system may perform a function for measuring an exercise amount on the basis of a sensor module included in the exercise amount measurement device. For example, an exercise amount measurement device may measure an exercise amount that is actually performed during a specific exercise on the basis of a physical quantities and/or measur ements (for example, pressure, location, distance, time, or speed) corresponding to sens or information measured through a sensor module.

In operation 1070, an exercise information providing system may perfor m a function for providing exercise information. According to an embodiment of the pr esent disclosure, an exercise information providing device included in an exercise infor mation providing system may provide a muscle specific exercise amount to a user (e.g., a breakdown according to respective muscle-groups) on the basis of an exercise portio n determined from an exercise portion determination device and an exercise amount me asured from an exercise amount measurement device. Additionally, an exercise informa tion providing device may provide user's body information or exercise detail informatio n to a user.

According to various embodiments of the present disclosure, an exercise information providing device may differently provide information corresponding to an exercise status or an exercise result by using user's body information or exercise histor y information collected from a storage module included in the exercise information pro viding device or an external electronic device connected to the exercise information pro viding device through a communication module on the basis of user identification infor mation. For example, an exercise information providing device may calculate a weight value for an exercise utilized for a user on the basis of exercise list information appropr iate for a user determined using user's body information or exercise history information . In this case, an exercise information providing device may differently display the displ ay location, form, transparency, or color of an object corresponding to information such as a muscle specific exercise amount measured during a specific exercise according to a weight value for an exercise. Additionally, an exercise information providing device may differently output the volume level or repeat count of voice information correspon ding to an exercise status or an exercise result as soon as an exercise is performed or ter minated.

In operation 1090, an exercise information providing system may perfor m a "check out" operation. According to an embodiment of the present disclosure, whe n a user terminates a specific exercise or terminates the use of the electronic devices inc luded in an exercise information providing system, the related electronic device may pe rform a check out operation. According to various embodiments of the present disclosu re, when a user performs a specific exercise provided from an exercise information pro viding system, the exercise information providing system may determine whether to ter minate the specific exercise on the basis of an exercise method, an exercise posture, an exercise time, or an exercise frequency according to the specific exercise provided from the exercise information providing system. Alternatively, when the use of an exercise d evice or an exercise auxiliary device including an exercise amount measurement device or connected to the exercise amount measurement device through a communication mo dule is terminated during a specific exercise, an exercise information providing system may determine the termination of the specific exercise. According to an embodiment of the present disclosure, when sensor information is maintained constantly because a use r does not exercise for a predetermined time, an exercise information providing system may determine the termination of a specific exercise through a sensor module included in an exercise amount measurement device.

In relation to the checkout operation, an exercise information providing system may store information generated on the basis of a result according to a function performance of the electronic devices in a storage module included in the electronic de vices. For example, information such as an exercise portion, an exercise amount, or a m uscle specific exercise amount according to an exercise performed by a user may be sto red in a storage module of the related electronic devices on the basis of user identificati on information. Additionally, the check-out operation may include an operation for dea ctivating the electronic devices.

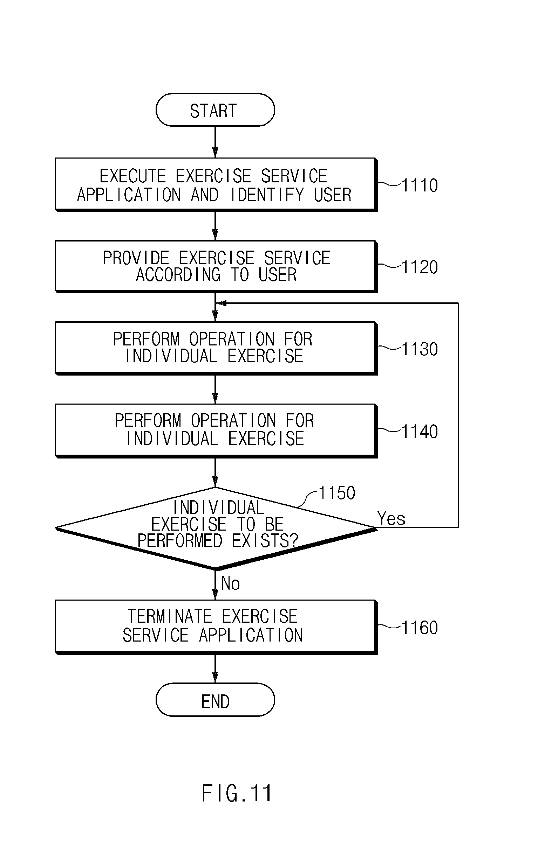

FIG. 11 is a view illustrating a method of operating an exercise informat ion providing system relating to exercise service according to various embodiments of t he present disclosure. In the description below, content identical, similar, or correspond ing to the above-mentioned content may be omitted below.

Referring to FIG. 11, in an exercise service providing method, in operati on 1110, an exercise information providing system (for example, the exercise informati on providing system 100 of FIG. 1) may perform exercise service application execution and a user identification operation. According to an embodiment of the present disclos ure, an exercise information providing system (for example, the exercise information pr oviding device 150 of FIG. 1) included in an exercise information providing system ma y include an exercise service application. When a user executes an exercise service appl ication included in an exercise information providing device, the exercise information p roviding device may identify users through a user identification module. Additionally, a n exercise information providing device may collect the identified user's body informat ion from a storage module included in the exercise information providing device or an external electronic device connected to the exercise information providing device throu gh wired/wireless communication.

According to an embodiment of the present disclosure, in the case of an IoT based exercise information providing system (for example, the exercise informatio n providing system 300 of FIG. 3), when a user enters or approaches a specific region ( for example, a service area managed by an IoT platform) where an IoT platform (for ex ample, the IoT internet platform 370 of FIG. 3) is located or IoT devices (for example, t he exercise portion determination device 310, the exercise amount measurement device 330, or the exercise information providing device 350 of FIG. 3) connected to the IoT platform through a communication module are located, the IoT platform may activate a n exercise information providing device (for example, the exercise information providi ng device 350 of FIG. 3) included in an exercise information providing system. In relati on to this, the IoT platform may identify the locations of users and the IoT devices on t he basis of LBS. Alternatively, a user's approach may be obtained based on a proximity sensor included in the IoT devices. According to an embodiment of the present disclos ure, an IoT platform may identify a user's location or a state that a user enters or approa ches the specific region on the basis of a communication module or a sensor module of an electronic device possessed or worn by a user.

According to various embodiments of the present disclosure, in relation t o an exercise service provided to a user, an IoT platform may perform a control to auto matically execute an exercise service application for performing an exercise service pro viding function included in an exercise information providing device. In this case, a use r may receive exercise list information appropriate for a user in time from an IoT platfo rm. Additionally, an IoT platform may perform a control to identify a user through a us er identification module included in an exercise information providing device.

When a user identification is completed, in operation 1120, an exercise i nformation providing system may provide an exercise service according to a user. Acco rding to an embodiment of the present disclosure, an exercise information providing sys tem may determine an exercise appropriate for a user on the basis of user's body inform ation or a preference for a specific exercise, which is included in user specific integrate d information, and then may provide it. The user specific integrated information may be information where user's body information, exercise history information, exercise deta il information, and a preference for specific exercise are integrated on the basis of user i dentification information. The user specific integrated information may be information generated based on information stored in a storage module of electronic devices include d in an exercise information providing system, or stored in an external electronic device connected through wired/wireless communication. According to an embodiment of the present disclosure, in the case of an IoT based exercise information providing system, t he user specific integrated information may be information stored in an external storage device (for example, the database 390 of FIG. 3) in an exercise service function.

In operation 1130, in relation to an exercise service provided from an ex ercise information providing system, a user may perform an individual exercise accordi ng to an exercise order corresponding to exercise list information. A step performance f or the individual exercise may be similar or identical to the above-mentioned operation in FIG. 10.

When the step performance for the individual step is terminated, in oper ation 1140, an exercise information providing system may output comprehensive exerci se information such as information on an individual exercise performance result, a prog ressed exercise situation, and an exercise to be performed. According to an embodimen t of the present disclosure, an exercise information providing system may output an exe rcise portion specific exercise amount according to an individual exercise performance or a currently processed exercise portion specific exercise amount. Additionally, an exe rcise information providing system may determine an exercise portion specific exercise amount remaining value on the basis of a currently progressed exercise portion specific exercise amount and an exercise portion specific exercise amount target value. In this c ase, an exercise information providing system may output exercise information relating to an individual exercise to be performed based on an exercise portion specific exercis e amount remaining value.

In operation 1150, an exercise information providing system may deter mine whether there is an individual exercise to be performed. If there is an individual e xercise to be performed, an exercise information providing system may return to operat ion 1130.

If there is no individual exercise to be performed, in operation 1160, an exercise information providing system may terminate an exercise service application. A ccording to an embodiment of the present disclosure, in the case of an IoT based exerci se information providing system, when a user is out of a region where an IoT platform i s located or a specific region (for example, a service area that an IoT platform manages ) where IoT devices connected to the IoT internet platform through a communication m odule are located, the IoT platform may deactivate an exercise information providing d evice included in an exercise information providing system. Additionally, an exercise s ervice application included in an exercise information providing device may be termina ted automatically. According to various embodiments of the present disclosure, in relati on to an exercise service application termination operation, an exercise information pro viding system may store comprehensive information on an exercise performed by a use r in a storage module or an external electronic device connected through a communicati on module.

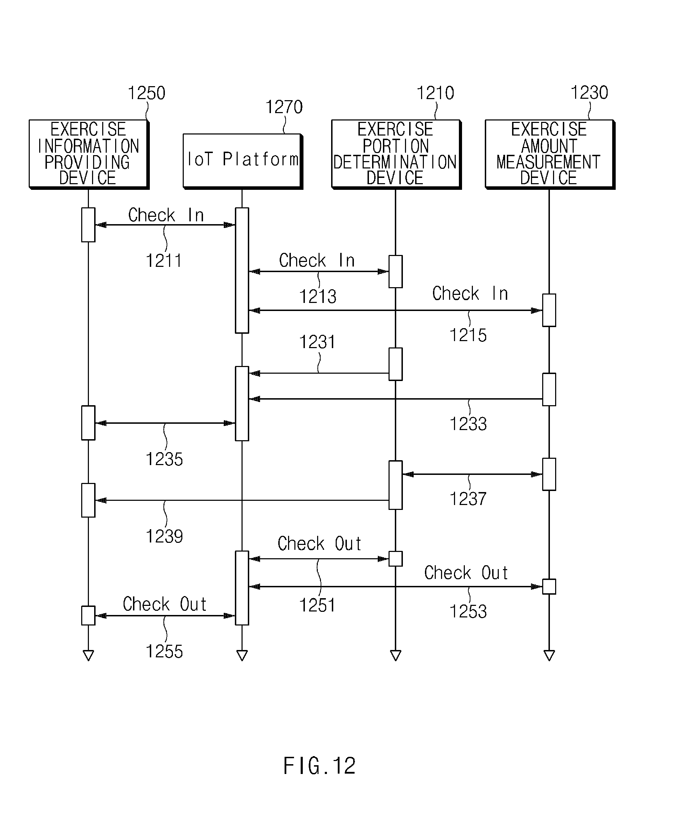

FIG. 12 is a view illustrating a method of operating an IoT based exercis e information providing system according to various embodiments of the present disclo sure.

Referring to FIG. 12, in relation to an IoT based exercise service providi ng method, an exercise information providing system may include an exercise portion d etermination device 1210, an exercise amount measurement device 1230, an exercise in formation providing device 1250, and an IoT platform 1270. According to an embodim ent of the present disclosure, when a user enters or approaches or moves into a specific geographic region (for example, a service area managed by the IoT platform 1270) whe re the IoT internet platform 1270 is located, or where IoT devices (for example, the exe rcise portion determination device 1210, the exercise amount measurement device 1230 , or the exercise information providing device 1250), connected to the IoT platform 127 0 through a communication module, are located, the IoT platform 1270 may activate th e exercise information providing device 1250 included in an exercise information provi ding system. Additionally, the IoT platform 1270 may perform a control to allow the ex ercise information providing device 1250 to identify a user. In this case, the exercise inf ormation providing device 1250 may perform a "check in" operation 1211 utilized user identification information corresponding to a user. For example, the exercise informatio n providing device 1250 may deliver user identification information to the IoT platform 1270.

According to various embodiments of the present disclosure, in relation t o the performance of the check in operation 1211, the IoT platform 1270 may collect us er specific integrated information from a storage module included in the IoT platform 1 270 or an external storage device (for example, the database 390 of FIG. 3) connected t o the IoT platform 1270 through a communication module in the basis of user identifica tion information. Additionally, the IoT platform 1270 may deliver the collected informa tion or exercise information appropriate for a user determined based on the collected inf ormation to the exercise information providing device 1250. In this case, the exercise in formation providing device 1250 may provide exercise service such as user's body info rmation, user's exercise history information, exercise detail information, and exercise li st information to a user on the basis of the information.

When a user approaches an exercise device or an exercise auxiliary devi ce relating to a specific exercise on the basis of the provided exercise service, the exerci se portion determination device 1210 and the exercise amount measurement device 123 0 relating to the specific exercise may also perform check in operations 1213 and 1215, respectively. In this case, the exercise portion determination device 1210 and the exerc ise amount measurement device 1230 may deliver exercise device information or exerci se auxiliary device information to the IoT platform 1270. Additionally, the exercise por tion determination device 1210 and the exercise amount measurement device 1230 may identify a user through a user identification module and may deliver the identified user identification information to the IoT platform 1270. The IoT platform 1270 may collect user specific integrated information on the basis of user identification information to d eliver it to the exercise portion determination device 1210 and the exercise amount mea surement device 1230, and the exercise portion determination device 1210 and the exer cise amount measurement device 1230 may use the user specific integrated information in order to perform functions thereof. According to various embodiments of the presen t disclosure, the exercise portion determination device 1210 or the exercise amount mea surement device 1230 may collect related exercise device information or exercise auxili ary device information from an external storage device connected to the IoT platform 1 270 through a communication module.

When a user performs a specific exercise, the exercise portion determina tion device 1210 may determine an exercise portion and perform operation 1231 for del ivering the determined information to the IoT platform 1270. Additionally, the exercise amount measurement device 1230 may measure an exercise amount and may perform operation 1233 for delivering the measured information to the IoT platform 1270. In thi s case, the IoT platform 1270 may perform operation 1235 for exchanging the informati on, or integrated or determined information, with the exercise information providing de vice 1250 based on the information. For example, the IoT platform 1270 may deliver a n exercise portion and an exercise amount to the exercise information providing device 1250 and the exercise information providing device 1250 may deliver at least one of int egrated information or exercise detail information, such as a muscle specific exercise a mount determined based on the integrated information, to the IoT platform 1270.

According to various embodiments of the present disclosure, an operatio n for delivering to the IoT platform 1270 at least one of an exercise portion determined through the exercise portion determination device 1210 and an exercise amount measur ed through the exercise amount measurement device 1230 may be omitted and the at le ast one may be directly delivered to the exercise information providing device 1250. Al ternatively, the exercise portion determination device 1210 and the exercise amount me asurement device 1230 may be connected through wired/wireless communication to per form operation 1237 for exchanging an exercise portion and an exercise amount with ea ch other and may perform operation 1239 for delivering the information to the exercise information providing device 1250.

According to various embodiments of the present disclosure, the exercis e information providing device 1250 may determine exercise detail information such as a muscle specific exercise amount on the basis of an exercise portion and an exercise a mount and may provide the exercise detail information to a user. Additionally, the exer cise information providing device 1250 may collect list information of exercise to be pe rformed by a user from the IoT platform 1270. For example, the exercise information p roviding device 1250 may collect information corresponding to the type of an exercise t o be performed by a user, an exercise name, an exercise intensity, an exercise frequenc y, or an exercise order. Additionally, the exercise information providing device 1250 m ay collect detail information on a related exercise, for example, information correspond ing to an exercise method, an exercise posture, or an exercise effect, according to an ex ercise order from the IoT platform 1270 or an external electronic device connected thro ugh wired/wireless communication, and may provide the collected information to a use r.

According to various embodiments of the present disclosure, when a rela ted exercise is terminated, the exercise portion determination device 1210 and the exerc ise amount measurement device 1230 may perform check out operations 1251 and 125 3. According to an embodiment of the present disclosure, in relation to an exercise serv ice provision, in the case that a specific exercise is newly provided to a user through the exercise information providing device 1250, if a user approaches an exercise device rel ating to a specific exercise, use an exercise device relating to a specific exercise, or wea rs an exercise auxiliary device relating to a specific exercise, the exercise portion deter mination device 1210 and the exercise amount measurement device 1230 correspondin g to the specific exercise may perform check in operations 1213 and 1215.

According to various embodiments of the present disclosure, in relation t o an exercise service provision, when all exercises provided are performed or exercise s ervice is terminated (for example, an exercise service application is terminated), the exe rcise information providing device 1250 may perform check out operation 1255. Accor ding to an embodiment of the present disclosure, when check out operation 1255 is perf ormed, the exercise information providing device 1250 may deliver user specific integr ated information to the IoT platform 1270 and store it. In this case, the IoT platform 12 70 may store user specific integrated information in a storage module included in the Io T platform 1270 or an external storage device connected to the IoT platform 1270 throu gh wired/wireless communication.

As mentioned above, an IoT based exercise information providing syste m may timely provide list information of exercises appropriate for a user or detail infor mation of an exercise to be performed by a user without a user's explicit instruction. Ac cording to an embodiment of the present disclosure, a user may use a fitness center pro viding a user specific exercise service while carrying a smartphone having an exercise s ervice application installed. In this case, when a user carrying a smartphone enters a fit ness center, the server may recognize the smartphone. In relation to this, the server may be an IoT platform and the smartphone may be an exercise information providing devi ce. The server may recognize that the smartphone enters a specific region where the ser ver is located, for example, a service area managed by the server by identifying the loca tion of the smartphone on the basis of LBS.

If the server recognizes the smartphone, the server may execute an exerc ise service application installed on the smartphone. In relation to this, the smartphone m ay transmit the user identification information of the smartphone to the server. Addition ally, the server may transmit, to the smartphone, user specific integrated information st ored in a storage module included in the server or an external storage device connected to the server through wired/wireless communication, by using the user identification inf ormation. The user specific integrated information may be information where user's bo dy information, exercise history information, exercise detail information, and a preferen ce for specific exercise are integrated on the basis of user identification information. Ad ditionally, the IoT platform 370 may determine an exercise appropriate for a user on the basis of user's body information or a preference for a specific exercise, which is includ ed in user specific integrated information, and may provide the determined exercise to t he smartphone.

If information on the smartphone user is not stored in the server, the serv er may collect user's body information from a user through an exercise service applicati on installed at the smartphone and may store the user's body information together with the user identification information. Additionally, the server may determines list informa tion of exercises appropriate for a user on the basis of user's body information and may deliver the list information to the smartphone to provide it to a user. If the server does not collect user's body information, it may deliver exercise list information correspondi ng to an initial setting value to the smartphone.

When a user performs a corresponding individual exercise according to an exercise order on the basis of exercise list information provided from the server, an e xercise device or an auxiliary device relating to the corresponding individual exercise may transmit user's identification information to the server in correspondence to user's approach, use, or wearing. In relation to this, the exercise device may be an exercise po rtion determination device and the exercise auxiliary device may be an exercise amount measurement device. The exercise device or the exercise auxiliary device may identify a user on the basis of user identification information inputted from a user through an in put module, biometric information measured through a sensor module, and user identifi cation information received from an external electronic device through a communicatio n module.

If receiving user identification from the exercise device or the exercise a uxiliary device, the server may transmit, to the exercise device or the exercise auxiliary device, user specific integrated information stored in a storage module included in the s erver or an external storage device connected to the server through wired/wireless com munication. In this case, the exercise device or the exercise auxiliary device may use us er's body information included in the user specific integrated information in order to pe rform a function.

In relation to an individual exercise performance, the smartphone may re ceive the determined exercise portion information and measured exercise amount infor mation from the exercise device or the exercise auxiliary device. Additionally, as soon as a corresponding individual exercise is performed or terminated, the smartphone may provide, to a user, exercise detail information such as an exercise portion, an exercise a mount, an exercise portion specific exercise amount, an exercise type, an exercise name , an exercise intensity, an exercise frequency, an exercise time, calorie consumption am ount, an exercise portion specific exercise amount target value, or an exercise portion s pecific exercise amount remaining value corresponding to a performed exercise. Additi onally, the smartphone may perform processing to transmit the exercise detail informati on to the server and store it.

When a user completes the performance of all individual exercises provi ded according to an exercise order on the basis of exercise list information provided fro m the server, the server may determine an inadequate part in an individual exercise that a user performs on the basis of an exercise portion specific exercise amount remaining value in the exercise detail information received from the smartphone and allow a user t o perform the inadequate part again. Additionally, if all exercises provided are terminat ed, the server may deliver overall results of exercises performed by a user to the smartp hone to deliver them to a user.

When a user carrying the smartphone leaves a fitness center, as the smar tphone leaves a specific region where the server is located, for example, a service area managed by the server, the server may store user specific integrated information in a st orage module included in the server or an external storage device connected to the serv er through wired/wireless communication. According to various embodiments of the pr esent disclosure, the server may deliver information on at what point which exercise is t o be performed better, that is, an exercise point and exercise list information, to the sma rtphone to provide it to a user.

According to various embodiments of the present disclosure, an exercise information providing method may include: receiving exercise portion information fro m a first electronic device; receiving exercise amount information from a second electr onic device; determining exercise information corresponding to an exercise portion spe cific exercise amount on the basis of at least the exercise portion information and the ex ercise amount information; and outputting the exercise information.

According to various embodiments of the present disclosure, the method may further include: outputting, by the first electronic device, the exercise information; or outputting, by the second electronic device, the exercise information.

According to various embodiments of the present disclosure, the receivi ng of the exercise portion information may include determining the exercise portion inf ormation on the basis of at least one of user input information, specified information, in formation received through at least one sensor included in the first electronic device, an d information received through at least one sensor included in the second electronic dev ice.

According to various embodiments of the present disclosure, the determi ning of the exercise portion information may further includes: including exercise portio n information corresponding to at least one of exercise place information, exercise devi ce information, and exercise auxiliary device information in the specified information; and storing the specified information in at least one of a storage module included in the first electronic device and an external storage device connected through wired/wireless communication.

According to various embodiments of the present disclosure, the determi ning of the exercise portion information may further include: performing a determinatio n on the basis of at least one of the information received through at least one sensor incl uded in the first electronic device and the information received through at least one sen sor included in the second electronic device and pattern information according to an op eration of a specified exercise method; and storing the pattern information in at least on e of a storage module included in the first electronic device and an external storage devi ce connected through wired/wireless communication.

According to various embodiments of the present disclosure, the receivi ng of the exercise amount information may include measuring the exercise amount info rmation on the basis of at least one of the information received through at least one sens or included in the first electronic device and the information received through at least o ne sensor included in the second electronic device.

According to various embodiments of the present disclosure, the determi ning of the exercise information may include: Including at least one of user's age, sex, weight, height, blood pressure, heart rate, muscle volume, body fat, and body mass inde x in the user's body information; Including at least one of the exercise portion correspo nding to an exercise performed, the exercise amount, an exercise portion specific exerci se amount determined based on the exercise portion and the exercise amount, an exerci se type, an exercise name, an exercise intensity, an exercise frequency, an exercise time , calorie consumption amount measured on the basis of the user's body information and the exercise amount, an exercise portion specific exercise amount target value, and an exercise portion specific exercise amount remaining value in the exercise detail informa tion; Including at least one of an exercise type, an exercise name, an exercise intensity, an exercise frequency, an exercise time, and an exercise order, each of which correspon ds to an exercise to be performed, in the exercise list information; and Including at least one of the user's body information, the exercise detail information, and the exercise lis t information in the exercise information.

According to various embodiments of the present disclosure, the method may further include: connecting the first electronic device, the second electronic devic e, and the electronic device, which are Internet of Things (IoT) devices, to an IoT platfo rm through wired/wireless communication; generating user specific integrated informat ion identified by each user on the basis of the exercise portion information received fro m the first electronic device, the exercise amount information received from the second electronic device, and the exercise information received from the electronic device; an d storing the generated user specific integrated information.

According to various embodiments of the present disclosure, the connect ing of the first electronic device, the second electronic device, and the electronic device may further include connecting an external storage device storing at least one of exerci se place information, exercise device information, exercise auxiliary device information , and the user specific integrated information to the IoT platform through wired/wireles s communication..

According to various embodiments of the present disclosure, the outputti ng of the exercise information may further include outputting at least one of voice infor mation corresponding to the exercise information and an object including a text or imag e corresponding to the exercise information.

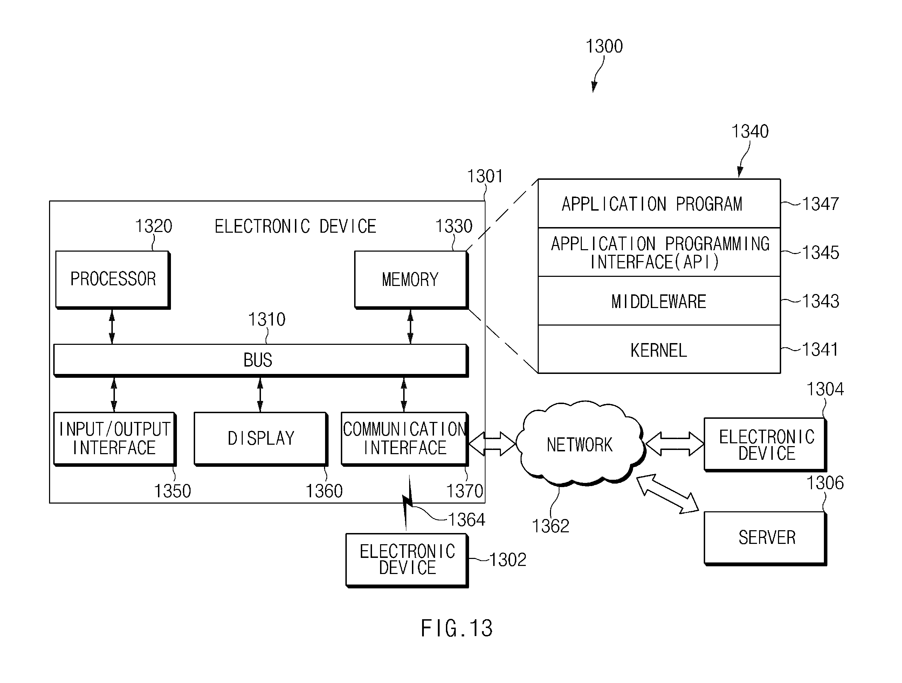

FIG. 13 is a view illustrating an electronic device in a network environm ent according to various embodiments of the present disclosure.

Referring to FIG. 13, an electronic device 1301 in a network environme nt 1300 is described according to various embodiments of the present disclosure. The el ectronic device 1301 may include a bus 1310, a processor 1320, a memory 1330, an inp ut/output interface 1350, a display 1360, and a communication interface 1370. Accordi ng to an embodiment of the present disclosure, the electronic device 1301 may omit at l east one of the components or may additionally include a different component.

The bus 1310, for example, may include a circuit for connecting the com ponents 1310 to 1370 to each other and delivering a communication (for example, contr ol message and/or data) therebetween.

The processor 1320 may include at least one of a central processing unit (CPU), an Application Processor (AP), and a communication processor (CP). The proc essor 1320, for example, may execute calculation or data processing for control and/or communication of at least one another component of the electronic device 1301.

The memory 1330 may include volatile and/or nonvolatile memory. The memory 1330, for example, may store instructions or data relating to at least one anoth er component of the electronic device 1301. According to an embodiment of the presen t disclosure, the memory 1330 may store software and/or program 1340. The programs 1340 may include a kernel 1341, a middleware 1343, an application programming inter face (API) 1345, and/or an application program (or an application) 1347. At least part o f the kernel 1341, the middleware 1343, or the API 1345 may be called an operating sy stem (OS).

The kernel 1341, for example, may control or manage system resources (for example, the bus 1310, the processor 1320, the memory 1330, and so on) used for performing operations or functions implemented in other programs (for example, the m iddleware 1343, the API 1345, or the application program 1347). Additionally, the kern el 1341 may provide an interface for controlling or managing system resources by acce ssing an individual component of the electronic device 1301 from the middleware 1343 , the API 1345, or the application program 1347.

The middleware 1343, for example, may serve as an intermediary role fo r exchanging data as the API 1345 or the application program 1347 communicates with the kernel 1341.

Additionally, the middleware 1343 may process at least one job request received from the application program 1347 according to a priority. For example, the m iddleware 1343 may assign to at least one application program 1347 a priority for using a system resource (for example, the bus 1310, the processor 1320, or the memory 1330 ) of the electronic device 1301. For example, the middleware 1343 may perform schedu ling or load balancing on the at least one job request by processing the at least one job r equest according to the priority assigned to the at least one job request.

The API 1345, as an interface for allowing the application program 1347 to control a function provided from the kernel 1341 or the middleware 1343, may inclu de at least one interface or function (for example, an instruction) for file control, windo w control, image processing, or character control.

The input/output interface 1350, for example, may serve as an interface f or delivering instructions or data inputted from a user or another external device to anot her component(s) of the electronic device 1301. Additionally, the input/output interface 1350 may output instructions or data received from another component(s) of the electr onic device 1301 to a user or another external device.

The display 1360, for example, may include a liquid crystal display (LC D), a light emitting diode (LED) display, an organic light emitting diode (OLED) displa y, a microelectromechanical systems (MEMS) display, or an electronic paper display. T he display 1360 may display various content (for example, text, image, video, icon, sy mbol, and so on) to a user. The display 1360 may include a touch screen, and for exam ple, may receive a touch, gesture, proximity, or hovering input by using an electronic p en or a user's body part.

The communication interface 1370, for example, may set a communicati on between the electronic device 1301 and an external device (for example, the first ext ernal electronic device 1302, the second external electronic device 1304, or the server 1 306). For example, the communication interface 1370 may communicate with an extern al device (for example, the second external electronic device 1304 or the server 1306) i n connection to the network 1362 through wireless communication or wired communic ation.

The wireless communication may use at least one of LTE, LTE-A, CDM A, WCDMA, UMTS, WiBro, or GSM as a cellular communication protocol, for examp le. Additionally, the wireless communication, for example, may include a short-range c ommunication 1364. The short range communication 1364, for example, may include a t least one of wireless fidelity (WiFi), Bluetooth (BT), near field communication (NFC) , global positioning system (GPS), and so on. The wired communication, for example, may include at least one of universal serial bus (USB), high definition multimedia inter face (HDMI), recommended standard 232 (RS-232), and plain old telephone service (P OTS). The network 1362 may include telecommunications network, for example, at lea st one of computer network (for example, LAN or WAN), internet, and telephone netw ork.

Each of the first and second external electronic devices 1302 and 1304 may be the same or different type of the electronic device 1301. According to an embo diment of the present disclosure, the server 1306 may include a group of one or more se rvers. According to various embodiments of the present disclosure, all or part of operati ons executed on the electronic device 1301 may be executed on another one or more ele ctronic devices (for example, the electronic device 1302 or 1304 or the server 1306). A ccording to an embodiment of the present disclosure, when the electronic device 1301 p erforms a certain function or service automatically or by a request, it may request at lea st part of a function relating thereto from another device (for example, the electronic de vice 1302 or 1304 or the server 1306) instead of or in addition to executing the function or service by itself. The other electronic device (for example, the external electronic de vice 1302 or 1304 or the server 1306) may execute a requested function or an additiona l function and may deliver an execution result to the electronic device 1301. The electro nic device 1301 may provide the requested function or service as it is or by processing t he received result additionally. For this, for example, cloud computing, distributed com puting, or client-server computing technology may be used.

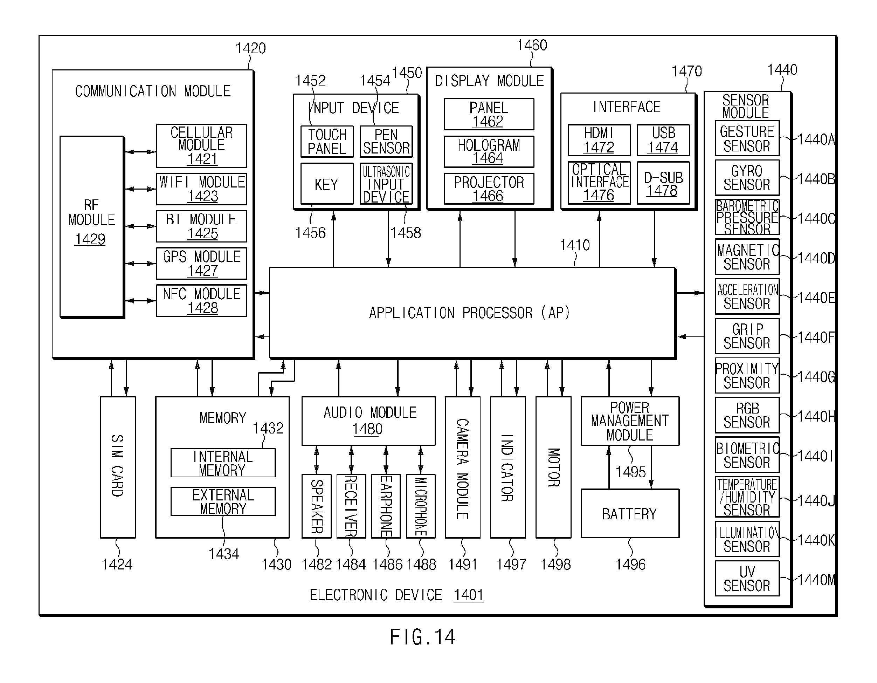

FIG. 14 is a block diagram of an electronic device according to various e mbodiments of the present disclosure.

Referring to FIG. 14, an electronic device 1401, for example, may confi gure all or part of the above-mentioned electronic device 1301 shown in FIG. 13. The e lectronic device 1401 may include at least one processor (for example, an application p rocessor (AP) 1410), a communication module 1420, a subscriber identification module (SIM) 1424, a memory 1430, a sensor module 1440, an input device 1450, a display 14 60, an interface 1470, an audio module 1480, a camera module 1491, a power manage ment module 1495, a battery 1496, an indicator 1497, and a motor 1498.

The processor 1410 may control a plurality of hardware or software com ponents connected thereto and also may perform various data processing and operations by executing an operating system or an application program. The processor 1410 may be implemented with a system on chip (SoC), for example. According to an embodime nt of the present disclosure, the processor 1410 may further include a graphic processin g unit (GPU) (not shown) and/or an image signal processor. The processor 1410 may in clude at least part (for example, the cellular module 1421) of components shown in FIG . 14. The processor 1410 may load commands or data received from at least one of othe r components (for example, nonvolatile memory) and process them and may store vario us data in a nonvolatile memory.

The communication module 1420 may have the same or similar configur ation to the communication interface 1370 of FIG. 13. The communication module 142 0 may include a cellular module 1421, a WiFi module 1423, a BT module 1425, a GPS module 1427, an NFC module 1428, and a radio frequency (RF) module 1429.

The cellular module 1421, for example, may provide voice call, video ca ll, text service, or internet service through communication network. According to an em bodiment of the present disclosure, the cellular module 1421 may perform a distinction and authentication operation on the electronic device 1401 in a communication network by using a SIM (for example, a SIM card) 1424. According to an embodiment of the pr esent disclosure, the cellular module 1421 may perform at least part of a function that t he processor 1410 provides. According to an embodiment of the present disclosure, the cellular module 1421 may further include a communication processor (CP).

Each of the WiFi module 1423, the BT module 1425, the GPS module 1 427, and the NFC module 1428 may include a processor for processing data transmitted /received through a corresponding module. According to an embodiment of the present disclosure, at least part (for example, at least one) of the cellular module 1421, the WiF i module 1423, the BT module 1425, the GPS module 1427, and the NFC module 1428 may be included in one integrated chip (IC) or IC package.

The RF module 1429, for example, may transmit/receive communicatio n signals (for example, RF signals). The RF module 1429, for example, may include a t ransceiver, a power amp module (PAM), a frequency filter, a low noise amplifier (LNA ), or an antenna. According to another embodiment of the present disclosure, at least on e of the cellular module 1421, the WiFi module 1423, the Bluetooth module 1425, the GPS module 1427, and the NFC module 1428 may transmit/receive RF signals through a separate RF module.

The SIM 1424, for example, may include a card including a SIM and/or an embedded SIM and also may include unique identification information (for example , an integrated circuit card identifier (ICCID) or subscriber information (for example, a n international mobile subscriber identity (IMSI).

The memory 1430 (for example, the memory 1330) may include an inter nal memory 1432 or an external memory 1434. The internal memory 1432 may include at least one of a volatile memory (for example, dynamic RAM (DRAM), static RAM ( SRAM), synchronous dynamic RAM (SDRAM) and a non-volatile memory (for examp le, one time programmable ROM (OTPROM), programmable ROM (PROM), erasable and programmable ROM (EPROM), electrically erasable and programmable ROM (EE PROM), mask ROM, flash ROM, flash memory (for example, NAND flash memory or NOR flash memory), hard drive, or solid state drive (SSD).

The external memory 1434 may further include flash drive, for example, compact flash (CF), secure digital (SD), micro Micro-SD, Mini-SD, extreme digital (x D), (MultiMediaCard or MMC), or a memorystick. The external memory 1434 may be functionally and/or physically connected to the electronic device 1401 through various i nterfaces.

The sensor module 1440 measures physical quantities or detects an oper ating state of the electronic device 1401, thereby converting the measured or detected i nformation into electrical signals. The sensor module 1440 may include at least one of a gesture sensor 1440A, a gyro sensor 1440B, a barometric pressure sensor 1440C, a m agnetic sensor 1440D, an acceleration sensor 1440E, a grip sensor 1440F, a proximity s ensor 1440G, a color sensor 1440H (for example, a red, green, blue (RGB) sensor), a bi ometric sensor 1440I, a temperature/humidity sensor 1440J, an illumination sensor 144 0K, and an ultra violet (UV) sensor 1440M. Additionally or alternatively, the sensor m odule 1440 may include an E-nose sensor, an electromyography (EMG) sensor, an elect roencephalogram (EEG) sensor, an electrocardiogram (ECG) sensor, an infra-red (IR) s ensor, an iris sensor, or a fingerprint sensor. The sensor module 1440 may further inclu de a control circuit for controlling at least one sensor therein. According to an embodim ent of the present disclosure, the electronic device 1401 may further include a processor configured to control the sensor module 1440 as part of or separately from the process or 1410 and thus may control the sensor module 1440 while the processor 1410 is in a s leep state.

The input device 1450 may include a touch panel 1452, a (digital) pen se nsor 1454, a key 1456, or an ultrasonic input device 1458. The touch panel 1452 may u se at least one of capacitive, resistive, infrared, or ultrasonic methods, for example. Add itionally, the touch panel 1452 may further include a control circuit. The touch panel 14 52 may further include a tactile layer to provide tactile response to a user.

The (digital) pen sensor 1454, for example, may include a sheet for reco gnition as part of a touch panel or a separate sheet for recognition. The key 1456 may i nclude a physical button, an optical key, or a keypad, for example. The ultrasonic input device 1458 may detect ultrasonic waves generated from an input tool through a microp hone (for example, the microphone 1488) in order to check data corresponding to the d etected ultrasonic waves.

The display 1460 (for example, the display 1360) may include a panel 1 462, a hologram device 1464, or a projector 1466. The panel 1462 may have the same o r similar configuration to the display 1360 of FIG. 13. The panel 1462 may be impleme nted to be flexible, transparent, or wearable, for example. The panel 1462 and the touch panel 1452 may be configured with one module. The hologram 1464 may show three-d imensional images in the air by using the interference of light. The projector 1466 may display an image by projecting light on a screen. The screen, for example, may be place d inside or outside the electronic device 1401. According to an embodiment of the pres ent disclosure, the display 1460 may further include a control circuit for controlling the panel 1462, the hologram device 1464, or the projector 1466.

The interface 1470 may include a high-definition multimedia interface ( HDMI) 1472, a universal serial bus (USB) 1474, an optical interface 1476, or a D-subm iniature (sub) 1478, for example. The interface 1470, for example, may be included in t he communication interface 1370 shown in FIG. 13. Additionally or alternatively, the i nterface 1470 may include a mobile high-definition link (MHL) interface, a secure Digi tal (SD) card/multi-media card (MMC) interface, or an infrared data association (IrDA) standard interface.

The audio module 1480 may convert sound into electrical signals and co nvert electrical signals into sounds. At least some components of the audio module 148 0, for example, may be included in the input/output interface 1350 shown in FIG. 13. T he audio module 1480 may process sound information inputted/outputted through a spe aker 1482, a receiver 1484, an earphone 1486, or a microphone 1488.

The camera module 1491, as a device for capturing a still image and a vi deo, may include at least one image sensor (for example, a front sensor or a rear sensor) , a lens (not shown), an image signal processor (ISP) (not shown), or a flash (not shown ) (for example, an LED or a xenon lamp).

The power management module 1495 may manage the power of the elec tronic device 1401. According to an embodiment of the present disclosure, the power m anagement module 1495 may include a power management IC (PMIC), a charger IC, or a battery or fuel gauge, for example. The PMIC may have a wired and/or wireless char ging method. As the wireless charging method, for example, there is a magnetic resona nce method, a magnetic induction method, or an electromagnetic method. An additional circuit for wireless charging, for example, a circuit such as a coil loop, a resonant circu it, or a rectifier circuit, may be added. The battery gauge may measure the remaining a mount of the battery 1496, or a voltage, current, or temperature thereof during charging . The battery 1496, for example, may include a rechargeable battery and/or a solar batte ry.

The indicator 1497 may display a specific state of the electronic device 1401 or part thereof (for example, the processor 1410), for example, a booting state, a message state, or a charging state. The motor 1498 may convert electrical signals into mechanical vibration and may generate vibration or haptic effect. Although not shown i n the drawings, the electronic device 1401 may include a processing device (for exampl e, a GPU) for mobile TV support. A processing device for mobile TV support may proc ess media data according to the standards such as digital multimedia broadcasting (DM B), digital video broadcasting (DVB), or mediaFLO.

Each of the above-mentioned components of the electronic device accor ding to various embodiments of the present disclosure may be configured with at least one component and the name of a corresponding component may vary according to the kind of an electronic device. According to various embodiments of the present disclosu re, an electronic device according to various embodiments of the present disclosure ma y include at least one of the above-mentioned components, may not include some of the above-mentioned components, or may further include another component. Additionall y, some of components in an electronic device according to various embodiments of the present disclosure are configured as one entity, so that functions of previous correspon ding components are performed identically.

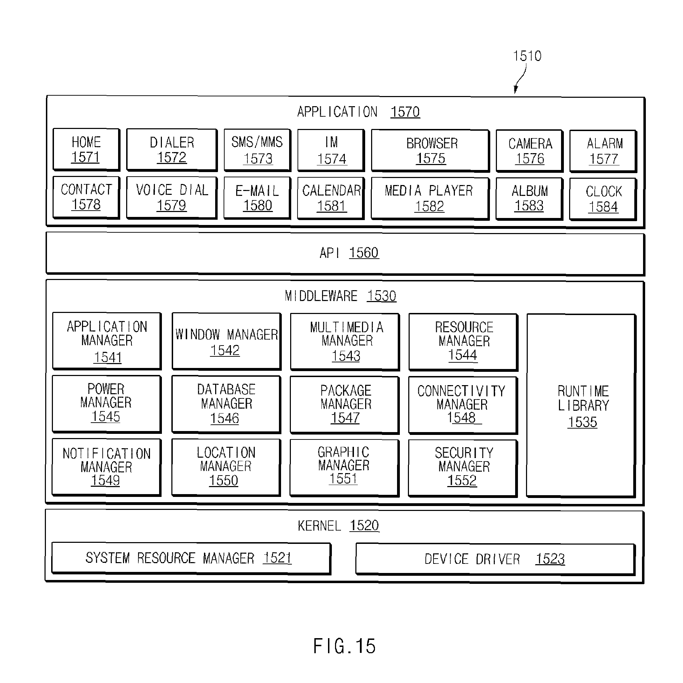

FIG. 15 is a block diagram of a program module according to various e mbodiments of the present disclosure.

According to an embodiment, the program module 1510 (for example, t he program 1340) may include an operating system (OS) for controlling a resource relat ing to an electronic device (for example, the electronic device 1301) and/or various app lications (for example, the application program 1347) running on the OS. The OS, for e xample, may include android, iOS, windows, symbian, tizen, or bada.

The program module 1510 may include a kernel 1520, a middleware 15 30, an API 1560, and/or an application 1570. At least part of the program module 1510 may be preloaded on an electronic device or may be downloaded from a server (for exa mple, the electronic devices 1302 and 1304 and the server device 1306).

The kernel 1520 (for example, the kernel 1341), for example, may inclu de a system resource manager 1521, or a device driver 1523. The system resource mana ger 1521 may perform the control, allocation, or retrieval of a system resource. Accordi ng to an embodiment of the disclosure, the system resource manager 1521 may include a process management unit, a memory management unit, or a file system management unit. The device driver 1523, for example, a display driver, a camera driver, a Bluetoot h driver, a sharing memory driver, a USB driver, a keypad driver, a WiFi driver, an aud io driver, or an inter-process communication (IPC) driver.

The middleware 1530, for example, may provide a function that the appl ication 1570 utilizes commonly, or may provide various functions to the application 15 70 through the API 1560 in order to allow the application 1570 to efficiently use a limit ed system resource inside the electronic device. According to an embodiment of the dis closure, the middleware 1530 (for example, the middleware 1343) may include at least one of a runtime library 1535, an application manager 1541, a window manager 1542, a multimedia manager 1543, a resource manager 1544, a power manager 1545, a databas e manager 1546, a package manager 1547, a connectivity manager 1548, a notification manager 1549, a location manager 1550, a graphic manager 1551, and a security manag er 1552.

The runtime library 1535, for example, may include a library module tha t a complier uses to add a new function through a programming language while the appl ication 1570 is running. The runtime library 1535 may perform a function on input/outp ut management, memory management, or an arithmetic function.

The application manager 1541, for example, may mange the life cycle of at least one application among the applications 1570. The window manager 1542 may manage a GUI resource used in a screen. The multimedia manager 1543 may recognize a format for playing various media files and may encode or decode a media file by usin g the codec corresponding to a corresponding format. The resource manager 1544 may manage a resource such as a source code, a memory, or a storage space of at least any o ne of the applications 1570.

The power manager 1545, for example, may operate together with a basi c input/output system (BIOS) to manage the battery or power and may provide power i nformation utilized for an operation of the electronic device. The database manager 154 6 may create, search, or modify a database used in at least one application among the a pplications 1570. The package manager 1547 may manage the installation or update of an application distributed in a package file format.

The connectivity manger 1548 may manage a wireless connection such as WiFi or Bluetooth. The notification manager 1549 may display or notify an event su ch as arrival messages, appointments, and proximity alerts to a user in a manner of not i nterrupting the user. The location manager 1550 may manage location information on a n electronic device. The graphic manager 1551 may manage a graphic effect to be provi ded to a user or a user interface relating thereto. The security manager 1552 may provid e various security functions utilized for system security or user authentication. Accordi ng to an embodiment, when an electronic device (for example, the electronic device 13 01) includes a phone function, the middleware 1530 may further include a telephony m anager for managing a voice or video call function of the electronic device.

The middleware 1530 may include a middleware module for forming a c ombination of various functions of the above-mentioned components. The middleware 1530 may provide a module specialized for each type of OS to provide differentiated fu nctions. Additionally, the middleware 1530 may delete part of existing components or a dd new components dynamically.

The API 1560 (for example, the API 1345), for example, as a set of API programming functions, may be provided as another configuration according to OS. Fo r example, in the case of android or iOS, one API set may be provided for each platfor m and in the case Tizen, at least two API sets may be provided for each platform.

The application 1570 (for example, the application program 1347) may i nclude at least one application for providing functions such as a home 1571, a dialer 15 72, an SMS/MMS 1573, an instant message 1574, a browser 1575, a camera 1576, an a larm 1577, a contact 1578, a voice dial 1579, an e-mail 1580, a calendar 1581, a media player 1582, an album 1583, a clock 1584, health care (for example, measure an exercis e amount or blood sugar), or environmental information provision (for example, provid e air pressure, humidity, or temperature information).

According to an embodiment, the application 1570 may include an appli cation (hereinafter referred to as "information exchange application") for supporting inf ormation exchange between the electronic device (for example, the electronic device 13 01) and an external electronic device (for example, the electronic devices 1302 and 130 4). The information exchange application, for example, may include a notification relay application for relaying specific information to the external device or a device manage ment application for managing the external electronic device.

For example, the notification relay application may have a function for r elaying to an external electronic device (for example, electronic devices 1302 and 1304 ) notification information occurring from another application (for example, an SMS/M MS application, an e-mail application, a health care application, or an environmental in formation application) of the electronic device. Additionally, the notification relay appl ication may receive notification information from an external electronic device and ma y then provide the received notification information to a user.

The device management application, for example, may manage (for exa mple, install, delete, or update) at least one function (turn-on/turn off of the external ele ctronic device itself (or some components) or the brightness (or resolution) adjustment of a display) of an external electronic device (for example, the electronic devices 1302 and 1304) communicating with the electronic device, an application operating in the ext ernal electronic device, or a service (for example, call service or message service) provi ded from the external device.

According to an embodiment of the disclosure, the application 1570 may include a specified application (for example, a health care application of a mobile medi cal device) according to the property of an external electronic device (for example, the electronic devices 1302 and 1304). According to an embodiment, the application 1570 may include an application received from an external electronic device (for example, th e server 1306 or the electronic device 1302 or 1304). According to an embodiment of t he disclosure, the application 1570 may include a preloaded application or a third party application downloadable from a server. The names of components in the program mod ule 1510 according to the shown embodiment may vary depending on the type of OS.

According to various embodiments of the present disclosure, at least part of the program module 1510 may be implemented with software, firmware, hardware, or a combination thereof. At least part of the programming module 1510, for example, may be implemented (for example, executed) by a processor (for example, the processo r 1410). At least part of the programming module 1510 may include a module, a progra m, a routine, sets of instructions, or a process to perform at least one function, for exam ple.

According to various embodiments of the present disclosure, by integrati ng and analyzing a variety of information in real time through collaboration between el ectronic devices, accurate information such as an exercise portion specific exercise amo unt may be provided.

Additionally, according to various embodiments of the present disclosur e, by using IoT, various and accurate information to which conditions appropriate for a user are applied may be provided in time.

The term "module" used in various embodiments of the present disclosu re, for example, may mean a unit including a combination of at least one of hardware, s oftware, and firmware. The term "module" and the term "unit", "logic", "logical block" , "component", or "circuit" may be interchangeably used. A "module" may be a minim um unit or part of an integrally configured component. A "module" may be a minimum unit performing at least one function or part thereof. A "module" may be implemented mechanically or electronically. For example, "module" according to various embodime nts of the present disclosure may include at least one of an application-specific integrat ed circuit (ASIC) chip performing certain operations, field-programmable gate arrays ( FPGAs), or a programmable-logic device, all of which are known or to be developed in the future.

According to various embodiments of the present disclosure, at least part of a device (for example, modules or functions thereof) or a method (for example, oper ations) according to this disclosure, for example, as in a form of a programming modul e, may be implemented using an instruction stored in computer-readable storage media. When at least one processor (for example, the processor 1320) executes an instruction, it may perform a function corresponding to the instruction. The non-transitory compute r-readable storage media may include the memory 1330, for example.

The non-transitory computer-readable storage media may include hard d isks, floppy disks, magnetic media (for example, magnetic tape), optical media (for exa mple, CD-ROM, and DVD), magneto-optical media (for example, floptical disk), and h ardware devices (for example, ROM, RAM, or flash memory). Additionally, a program instruction may include high-level language code executable by a computer using an in terpreter in addition to machine code created by a complier. The hardware device may be configured to operate as at least one software module to perform an operation of vari ous embodiments of the present disclosure and vice versa.

A module or a programming module according to various embodiments of the present disclosure may include at least one of the above-mentioned components, may not include some of the above-mentioned components, or may further include anot her component. Operations performed by a module, a programming module, or other co mponents according to various embodiments of the present disclosure may be executed through a sequential, parallel, repetitive or heuristic method. Additionally, some operati ons may be executed in a different order or may be omitted. Or, other operations may b e added.

Moreover, the embodiments disclosed in this specification are suggested for the description and understanding of technical content but do not limit the range of the present disclosure. Accordingly, the range of the present disclosure should be interp reted as including all modifications or various other embodiments based on the technica l idea of the present disclosure.

## Claims

1. An electronic device, comprising:
an output module;
a communication module configured to allow communication with at least one of a first electronic device and a second electronic device; and
at least one processor, configured to:
receive exercise portion information indicating at least a part of a muscle group affected by an exercise performed by a user from the first electronic device,
receive exercise amount information including at least one of pressure applied to a part of a muscle group, moving time, moving speed, and moving acceleration measured while a user performs an exercise from the second electronic device, and
determine exercise information including a respective amount of exercise for each exercise portion based on at least the received exercise portion information and the received exercise amount information, and
control the output module to output the exercise information.

2. The electronic device of claim 1, wherein the electronic device comprises the first electronic device or the second electronic device.

3. The electronic device of claim 1, wherein the exercise portion information is based on at least one of:
input information received from a user,
specified information indicating at least expected a part of a muscle group affected by an exercise performed by a user,
information generated by at least one sensor included in the first electronic device, and information generated by at least one sensor included in the second electronic device.

4. The electronic device of claim 3, wherein the specified information comprises exercise portion information corresponding to at least one of exercise place information indicating designation of a location where an exercise on a specified exercise type can be performed by a user, exercise device information, and exercise auxiliary device information, and is stored in at least one of a storage module included in the first electronic device and an external storage device communicatively connected through wired and/or wireless communication.

5. The electronic device of claim 3, wherein at least one of the information generated through the at least one sensor included in the first electronic device, and the information generated through the at least one sensor included in the second electronic device comprises:
pattern information indicating an operation of the first electronic device or second electronic device respectively during execution of an exercise method,
wherein the pattern information is stored in at least one of a storage module included in the first electronic device and second electronic device respectively, and an external storage device in communication with the electronic device through wired and/or wireless communication.

6. The electronic device of claim 1, wherein the second electronic device measures the exercise amount information through at least one of:
at least one sensor included in the first electronic device, and
at least one sensor included in the second electronic device.

7. The electronic device of claim 1, wherein the exercise information comprises at least one of: user's body information, comprising at least one of user's age, sex, weight, height, blood pressure, heart rate, muscle volume, body fat, and body mass index,
exercise detail information, comprising at least one of a portion of a user's body corresponding to an exercise performed, an exercise amount, a portion-specific exercise amount based on the portion of the user's body and the exercise amount, an exercise type, an exercise name, an exercise intensity, an exercise frequency, an exercise time, a caloric consumption amount based on the user's body information and the exercise amount, a target exercise amount value, and a remaining value to complete of the target exercise amount value, and
exercise list information comprises at least one of an exercise type, an exercise name, an exercise intensity, an exercise frequency, an exercise time, and an order of execution for a plurality of exercises, each corresponding to an exercise to be performed.

8. The electronic device of claim 1, wherein:
the first electronic device, the second electronic device, and the electronic device are Internet of Things (IoT) enabled devices communicatively connected to an IoT platform through wired and/or wireless communication; and
the IoT platform is configured to generate user-specific integrated information identified by each user based of the exercise portion information received from the first electronic device, the exercise amount information received from the second electronic device, and the exercise information determined the electronic device, and store the generated user-specific integrated information.

9. The electronic device of claim 8, wherein the IoT platform is communicatively connected to an external storage device storing at least one of exercise place information indicating designation of a location where an exercise on a specified exercise type can be performed by a user, exercise device information, exercise auxiliary device information, and the user specific integrated information, through wired and/or wireless communication.

10. The electronic device of claim 1, wherein the at least one processor controls the output module to output at least one of voice information corresponding to the exercise information, and at least one of a text and image corresponding to the determined exercise information.

11. An method in an electronic device, comprising:
receiving, via a communication module, exercise portion information indicating at least a part of a muscle group affected by an exercise performed by a user from a first electronic device;
receiving exercise amount information including at least one of pressure applied to a part of a muscle group, moving time, moving speed, and moving acceleration measured while a user performs an exercise from a second electronic device;
determining, by at least one processor, exercise information including a respective amount of exercise for each exercise portion based on at least the received exercise portion information and the received exercise amount information, and
controlling an output module to output the exercise information.

12. The method of claim 11, wherein the first electronic device determines the exercise portion information is based on at least one of:
input information received from a user,
specified information indicating at least expected a part of a muscle group affected by an exercise performed by a user,
information generated by at least one sensor included in the first electronic device, and
information generated by at least one sensor included in the second electronic device.

13. The method of claim 12, wherein the determining the exercise portion information further comprises:
determining the exercise portion information is based on the specified information corresponding to at least one of: exercise place information indicating designation of a location where an exercise on a specified exercise type can be performed by a user, exercise device information, and exercise auxiliary device information,
storing the specified information in at least one of a storage module included in the first electronic device and an external storage device communicatively connected through wired and/or wireless communication.
determining the exercise portion information is based on the information generated by at least one sensor included in the first electronic device or the second electronic device corresponding to a pattern information indicating an operation of the first electronic device or second electronic device respectively during execution of an exercise method, and
storing the pattern information in at least one of a storage module included in the first electronic device and second electronic device respectively, and an external storage device in communication with the electronic device through wired and/or wireless communication.

14. The method of claim 11, further comprising determining the exercise information is based on at least one of:
user's body information, comprising at least one of user's age, sex, weight, height, blood pressure, heart rate, muscle volume, body fat, and body mass index,
exercise detail information, comprising at least one of a portion of a user's body corresponding to an exercise performed, an exercise amount, a portion-specific exercise amount based on the portion of the user's body and the exercise amount, an exercise type, an exercise name, an exercise intensity, an exercise frequency, an exercise time, a caloric consumption amount based on the user's body information and the exercise amount, a a target exercise amount value, and a remaining value to complete of the target exercise amount value, and
exercise list information, comprising at least one of an exercise type, an exercise name, an exercise intensity, an exercise frequency, an exercise time, and an order of execution for a plurality of exercises, each corresponding to an exercise to be performed.

15. The method of claim 11, further comprising:
connecting the first electronic device, the second electronic device, and the electronic device are Internet of Things (IoT) enabled devices communicatively connected to an IoT platform through wired and/or wireless communication;
generating user-specific integrated information identified by each user based of the exercise portion information received from the first electronic device, the exercise amount information received from the second electronic device, and the exercise information determined by the electronic device; and
storing the generated user-specific integrated information.
